(19) Europäisches Patentamt
European Patent Office
Office européen des brevets

(11) Veröffentlichungsnummer: **0 018 510**
**B1**

(12) # EUROPÄISCHE PATENTSCHRIFT

(45) Veröffentlichungstag der Patentschrift: **21.12.83**

(21) Anmeldenummer: **80101863.1**

(22) Anmeldetag: **08.04.80**

(51) Int. Cl.³: **C 07 D 307/32**, C 07 D 249/08, C 07 C 103/34, C 07 C 143/00, C 07 D 231/12, C 07 D 405/12, A 01 N 37/22, A 01 N 43/08, A 01 N 43/56, A 01 N 43/64

(54) **N-Acylierte alpha-Naphthylamine, deren Herstellung und Verwendung als Pflanzenfungizide sowie sie enthaltende pflanzenfungizide Mittel.**

(30) Priorität: **10.04.79 CH 3404/79**

(43) Veröffentlichungstag der Anmeldung:
**12.11.80 Patentblatt 80/23**

(45) Bekanntmachung des Hinweises auf die Patenterteilung:
**21.12.83 Patentblatt 83/51**

(84) Benannte Vertragsstaaten:
**AT BE CH DE FR GB IT LI NL SE**

(56) Entgegenhaltungen:
**DE - A - 2 724 785**
**DE - A - 2 819 879**
**DE - B - 1 003 221**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Patentabteilung Postfach**
**CH-4002 Basel (CH)**

(72) Erfinder: **Moser, Hans, Dr.**
**Blumenrain 3**
**CH-4465 Magden (CH)**
Erfinder: **Eckhardt, Wolfgang, Dr.**
**Breslauerstrasse 16**
**D-7850 Lörrach (DE)**
Erfinder: **Kunz, Walter, Dr.**
**79 Gleneagles Road**
**Flixton Manchester M31 2SA (GB)**
Erfinder: **Hubele, Adolf, Dr.**
**Obere Egg 9**
**CH-4465 Magden (CH)**

(74) Vertreter: **Zumstein, Fritz sen., Dr. et al,**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 018 510 B1

N-Acylierte alpha-Naphthylamine, deren Herstellung und Verwendung als Pflanzenfungizide sowie sie enthaltende pflanzenfungizide Mittel.

Die Erfindung betrifft Verbindungen der Formel I

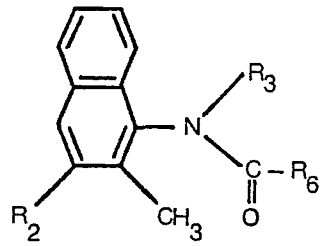

(I)

worin $R_2$ Wasserstoff oder Methyl bedeutet, $R_3$ für $-CH(CH_3)COOR_4$ oder

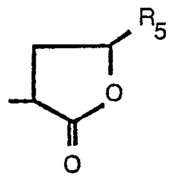

steht, wobei $R_4$ gegebenenfalls durch Halogen oder $C_1$—$C_2$-Alkoxy substituiertes $C_1$—$C_4$-Alkyl, $C_2$—$C_4$-Alkenyl, $C_3$—$C_4$-Alkinyl oder $C_3$—$C_7$-Cycloalkyl und $R_5$ Wasserstoff oder Methyl bedeuten und $R_6$ gegebenenfalls durch Halogen substituiertes 2-Furyl oder 2-Tetrahydrofuryl, $\beta$-($C_1$—$C_4$)-alkoxyäthyl oder die Gruppe $CH_2Z$ bedeutet, wobei Z für eine der Gruppen a) —X—$R_7$, b) —NH—N($R_8$) ($R_9$), c) $-OSO_2R_{10}$, d) —O(CO)$R_{11}$, e) 1,2-Pyrazolyl (1) oder, f) 1,2,4-Triazolyl (1) steht, unter Einschluss ihrer Salze und Metallkomplexe, und X Sauerstoff oder Schwefel ist, $R_7$ eine durch $C_1$—$C_2$-Alkoxy substituiertes $C_1$—$C_6$-Alkyl, oder aber $C_3$—$C_4$-Alkenyl oder $C_3$—$C_4$-Alkinyl bedeutet, $R_8$ Wasserstoff oder $C_1$—$C_3$-Alkyl, $R_9$ $C_1$—$C_3$-Alkyl oder Phenyl, $R_{10}$ $C_1$—$C_4$-Alkyl oder Mono- oder Di($C_1$—$C_3$)-Alkylamin und $R_{11}$ gegebenenfalls durch $C_1$—$C_2$-Alkoxy substituiertes $C_1$—$C_3$-Alkyl darstellen, wobei im Falle $R_3$ = $-CH(CH_3)COOR_4$ der Substituent $R_7$ auch $C_1$—$C_6$-Alkyl bedeuten kann.

Unter Alkyl oder als Alkylteil eines anderen Substituenten sind je nach Zahl der angegebenen C-Atome folgende Gruppen zu verstehen:
Methyl, Aethyl, Propyl, Butyl, Pentyl, Hexyl, sowie ihre Isomeren, wie z.B. iso-Propyl, iso-Butyl, sek-Butyl, tert-Butyl, iso-Pentyl. Alkenyl steht beispielsweise für Allyl oder 2-Butenyl. Alkinyl bedeutet vor allem Propargyl. Als $C_3$—$C_7$-Cycloalkyl sind Cyclopropyl, Cyclobutyl, Cyclopentyl, Cyclohexyl und Cycloheptyl zu verstehen. Halogen steht für Fluor, Chlor, Brom oder Jod.

Als Metallkationen in Komplexen von Verbindungen der Formel I werden vorzugsweise solche aus den Hauptgruppen II und IV sowie den Nebengruppen I, II und IV bis VIII des Periodensystems eingesetzt wie z.B. Mg, Ca, Ba, Sn, Pb, Cu, Zn, Cd, Cr, Mn, Fe, Co und Ni.

Als salzbindende Säuren für die Verbindungen der Formel (I) kommen solche mit guter Pflanzenverträglichkeit in Frage. Hierzu gehören die Halogenwasserstoffsäuren (wie z.B. die Chlorwasserstoffsäure und die Bromwasserstoffsäure) ferner die Schwefelsäure, Phosphorsäure, Salpetersäure, mono- und bifunktionelle Carbonsäuren und Hydroxycarbonsäuren, wie z.B. Essigsäure, Weinsäure, Citronensäure, Salicylsäure, Milchsäure, 1,5-Naphthalin-disulfonsäure, Methansulfonsäure und Benzolsulfonsäure.

Die Verbindungen der Formel I können wie nachfolgend unter A—G aufgeführt, nach einer ganzen Reihe von Methoden hergestellt werden. In den Formeln II bis XVIII haben $R_2$ bis $R_{11}$ und X die unter Formel I angegebenen Bedeutungen, Hal steht für Halogen, vorzugsweise Chlor oder Brom und M für Wasserstoff oder ein Metallvorzugsweise Alkali- oder Erdalkalikation.

A. 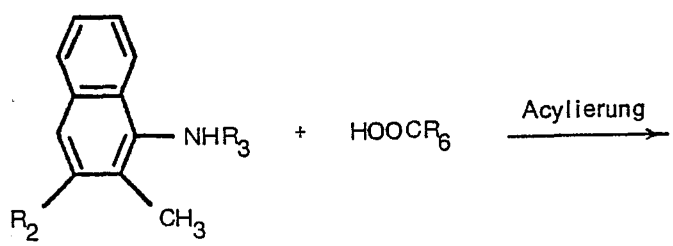


(I)

(II) (III)

Es kann vorzugsweise ein reaktionsfähiges Derivat der Carbonsäure der Formel III, wie z.B. das Säurehalogenid, Säureanhydrid oder der Ester eingesetzt werden. Als Säurehalogenid eignet sich vornehmlich das Säurechlorid oder Säurebromid.

In manchen Fällen ist die Verwendung von säurebindenden Mitteln bzw. Kondensationsmitteln vorteilhaft. Als solche kommen z.B. tertiäre Amine wie Trialkylamine (z.B. Triäthylamin), Pyridin und Pyridinbasen oder anorganische Basen, wie die Oxide, Hydroxide, Hydrogencarbonate, Carbonate oder Hydride von Alkali- und Erdalkalimetallen sowie Natriumacetat in Betracht. Als säurebindendes Mittel kann ausserdem das Ausgangsprodukt II dienen.

Das Herstellungsverfahren A kann auch ohne säurebindende Mittel durchgeführt werden, wobei in einigen Fällen das Durchleiten von Stickstoff zur Vertreibung des gebildeten Halogenwasserstoffs angezeigt ist. In anderen Fällen ist ein Zusatz von Dimethylformamid als Reaktionskatalysator sehr vorteilhaft.

B. Wenn $R_6$ $-CH_2OSO_2R_{10}$ bzw. $-CH_2-O(CO)R_{11}$ bedeutet, lässt sich nach einleitender Acylierung einer Verbindung der Formel II mit Hydroxyessigsäure (oder einem Derivat) zu Formel IV folgende Variante durchführen:

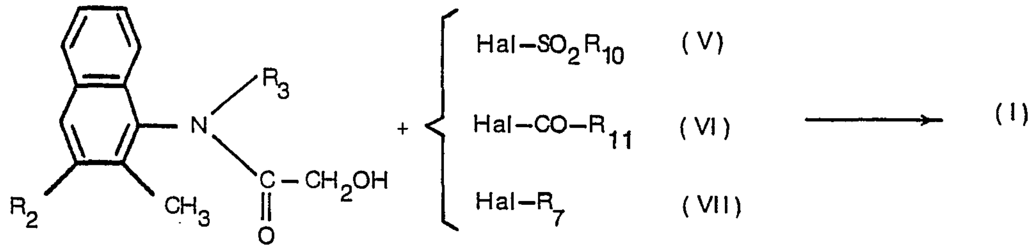

(IV)

Bei der Reaktionsvariante B wird vorteilhafterweise ein Salz (=Alkoholat), vor allem ein Alkalisalz der Verbindung der Formel IV, verwendet.

Dieses Verfahren wird, wenn erforderlich, in Gegenwart eines wie unter A beschriebenen säurebindenden Mittels durchgeführt.

C. Falls $R_6$ eine andere Bedeutung als $-CH_2NH-N(R_8)(R_9)$ hat, lässt sich auch folgende Verfahrensvariante durchführen:

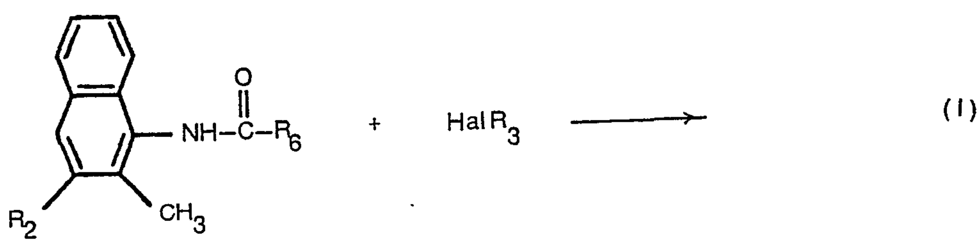

(VIII) (IX)

Hierbei wird die Verbindung der Formel VIII zuerst mit Butyl-Lithium oder Na-Hydrid in das entsprechende N-Alkalisalz überführt oder aber das Verfahren wird in Gegenwart eines säurebindenden Mittels analog Verfahren A, vorzugsweise unter Zusatz katalytischer Mengen Alkalijodid, durchgeführt.

D. Wenn $R_6$ für $-CH_2XR_7$; $-CH_2-O-CO-R_{11}$, $-CH_2NH-N(R_8)(R_9)$ oder ein Azolylmethyl steht (Azolyl = 1,2-Pyrazolyl (1) oder 1,2,4-Triazolyl (1)), lässt sich folgende Variante nach einleitender Haloacetylierung einer Verbindung der Formel II zu Formel X durchführen:

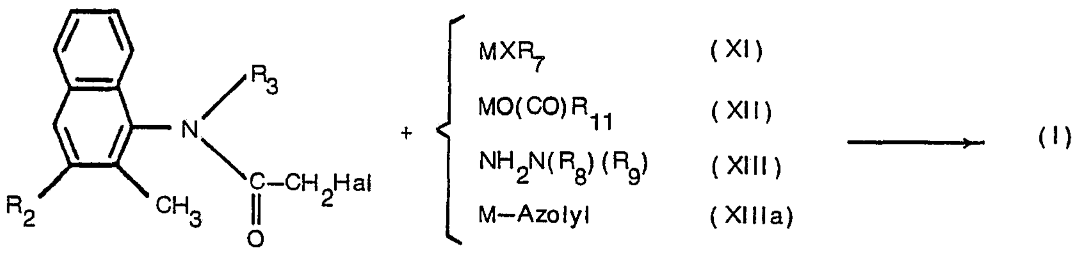

(X)

Wenn M=Wasserstoff bedeutet, ist die Verwendung eines salzbildenden Mittels angebracht, wie z.b. eines Oxids, Hydroxids, Hydrids von Alkali- oder Erdalkalimetallen, Bei Verwendung von Ausgangsstoffen der Formel XIII oder XIIIa wird das Endprodukt als Hydrohalogenid erhalten. Mit milden Basen lässt sich daraus bei Raumtemperatur oder bei leicht erhöhter Temperatur die freie Hydrazino- bzw. Azol-Base erhalten. Es eignen sich hierfür z.B. Alkalicarbonate.

E. Wenn $R_6$ für $\beta$-($C_1$—$C_4$)-Alkoxyäthyl steht:

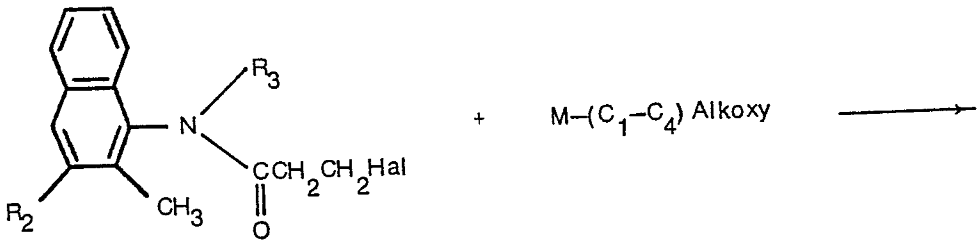


(I)

(XIV) (XV)

Hierbei wird analog zum Verfahren D gearbeitet.

F. Wenn $R_6$ für $\beta$-($C_1$—$C_4$)-Alkoxyäthyl steht:

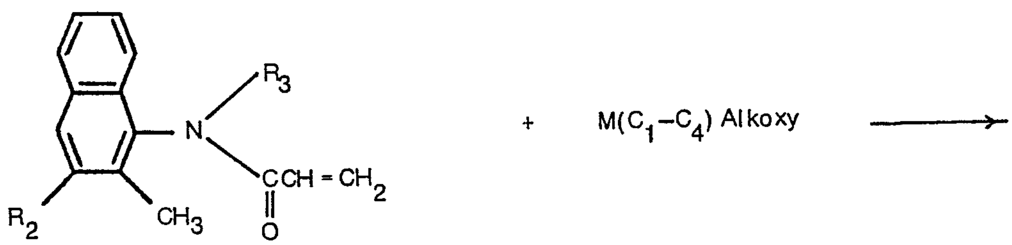


(I)

(XVI) (XV)

Bei diesem Verfahren wird mit dem alkohol bzw. mit dem Alkoholat XV (M=Metallatom) eine Michaeladdition durchgeführt.

G. Wenn $R_3$ für $—CH(CH_3)COOR_4$ steht, lässt sich als letzter Reaktionsschritt die Veresterung der Seitenkette des bereits gebildeten Acylamids durchführen:

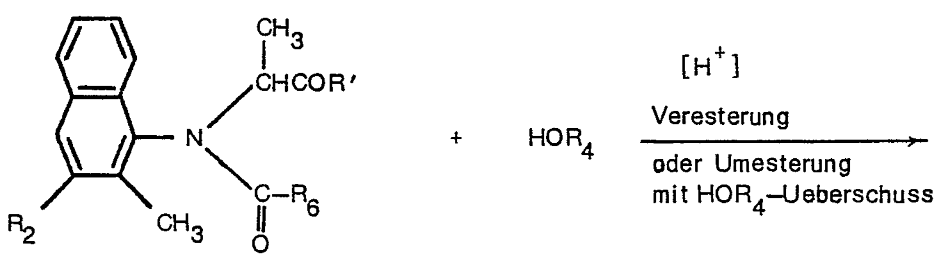


(I)

(XVII) (XVIII)

Hierbei bedeutet R′ wahlweise —OH oder einen anderen alkoholischen Rest $—OR'_4$, der nach üblichen Methoden ausgetauscht werden kann.

Bedeutet R′ dagegen ein Halogenatom, so wird die Veresterung zweckmässig in Gegenwart eines säurebindenden Mittels vorgenommen.

Bedeutet R′ = OMe, wobei Me eine Alkali-, Erdalkali-, Blei- oder Silbermetallatom darstellt, so lässt sich die Veresterung auch gemäss

$$XVII + Hal'—R_4 \rightarrow (I)$$

durchführen. Dabei stellt Hal′ ein Halogenatom, bevorzugt Chlor, Brom oder Jod dar.

Es können bei allen Verfahren Lösungsmittel verwendet werden, die den Reaktionsteilnehmern gegenüber inert sein müssen.

Beispiele solcher Lösungsmittel sind:

Aliphatische Kohlenwasserstoffe wie Benzol, Toluol, Xylole, Petroläther; halogenierte Kohlenwasserstoffe wie Chlorbenzol, Methylenchlorid, Aethylenchlorid, Chloroform; Aether und ätherartige Verbindungen wie Dialkyläther, Dioxan, Tetrahydrofuran; Nitrile wie Acetonitril; N,N-dialkylierte Amide

wie Dimethylformamid; Dimethylsulfoxid, Ketone wie Methyläthylketon und Gemische solcher Lösungsmittel untereinander.

Die verschiedenen Verfahren sind ebenfalls ein Teil der Erfindung.

Die Ausgangsstoffe sind zum Teil neu und zeigen gleichfalls eine fungizide Wirkung. Sie werden nach an sich bekannten Methoden hergestellt.

Die Verbindungen der Formel I besitzen in $R_3$ ein asymmetrisches Kohlenstoffatom und können auf übliche Art in optische Antipoden gespalten werden; so z.B. durch fraktionierte Kristallisation des Salzes, gebildet aus einer Verbindung der Formel II und einer optisch aktiven Säure, und Weiterreaktion des daraus erhaltenen optischen Antipoden von II zu einem Enantiomeren der Formel I. Fraktionierte Kristallisation eines Salzes aus XVII einer optisch aktiven Base und Weiterreaktion des wieder freigesetzten optischen Antipoden von XVII zu einem Enantiomeren der Formel I ist eine weitere Möglichkeit der Gewinnung optischer Antipoden der Formel I. Diese besitzen unterschiedliche mikrobizide Wirkungen.

Je nach Substitution können weitere asymmetrische Kohlenstoffatome im Molekül auftreten.

Sofern keine gezielte Synthese zur Isolierung reiner Isomerer durchgeführt wird, fällt normalerweise ein Produkt der Formel I als Diastereomeren-Gemisch an. Dies liefert bei getrennter Aufarbeitung Produkte mit unterschiedlichen physikalischen Kenndaten.

Die Verbindungen der Formel I können für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z.b. natürlichen oder regenerierten mineralischen Stoffen, Lösungs-, Dispergier-, Netz-, Haft-, Verdickungs-, Binde- oder Düngemitteln.

Der Gehalt an Wirkstoff in handelsfähigen Mitteln liegt zwischen 0,1 und 90%.

Zur Applikation können die Verbindungen der Formel I in den folgenden Aufarbeitungsformen vorliegen (wobei die Gewichts-Prozent-angaben in Klammern vorteilhafte Mengen an Wirkstoff darstellen): Feste Aufarbeitungsformen: Stäubemittel und Streumittel (bis zu 10%) Granulate, Umhüllungsgranulate, Imprägnierungsgranulate und Homogengranulate, Pellets (Körner) (1 bis 80%):

Flüssige Aufarbeitungsformen:

a) in Wasser dispergierbare Wirkstoffkonzentrate:

Spritzpulver (wettable powders) und Pasten (25—90% in der Handelspackung, 0,01 bis 15% in gebrauchsfertiger Lösung); Emulsions- und Lösungskonzentrate (10 bis 50%; 0,01 bis 15% in gebrauchsfertiger Lösung);

b) Lösungen (0,1 bis 20%); Aerosole.

Solche Mittel gehören ebenfalls zur Erfindung.

In der BE—PS 871,668 werden in allgemeiner Form Acetamide als Fungizide gennant, darunter auch vereinzelt solche, die sich vom $\alpha$-Naphthylamin ableiten. Ein typischer Vertreter, dieser Reihe, das N-(2-Methylnaphtyl)-N-(2-oxo-tetrahydrofuran-3-yl)-N-methoxy-acetylamin, wird in den wiedergegebenen Versuchen als wirkungslos gekennzeichnet, ganz im Gegensatz zu den fungizid wirksamen analogen Vertretern der Phenylamin-Reihe (=Anilinreihe). Diese Lehre der BE—PS 871,668 gibt dem Fachmann keine Hinweise auf die Existenz hochwirksamer Pflanzenfungsizide in der Reihe der acylierten $\alpha$-Naphthylamine der Formel I Erfindung. Es hat sich in dieser Reihe überraschend gezeigt, dass nur die Kombination bestimmter Strukturelemente zu neuartigen, hochwirksamen Pflanzenfungiziden führt, wie sie vorliegend mit der Formel I wiedergegeben werden, insbesondere die Kombination der für $R_6$ genannten Gruppen mit einer der beiden für $R_3$ genannten Seitenketten. Derartige Kombinationen führen zu besonders pflanzenverträglichen Fungiziden ohne störende Nebenwirkungen. Wirkstoffe der Formel I zeichnen sich ausserdem durch deutliche Dauerwirkung aus.

Demgemäss bezieht sich die Erfindung auf folgende Untergruppen von acylierten $\alpha$-Naphthylaminen:

a) $—CO—CH_2—X—R_7$: aliphatische Acylverbindungen,

b) $—CO—CH_2—NH—N(R_8)(R_9)$: Hydrazinoacetylderivate,

c) $—CO—CH_2—O—SO_2R_{10}$: Sulfonyl- und Sulfamoyl-acetylderivate,

d) $—CO—CH_2—O—COR_{11}$: Acylierte Hydroxyacetylderivate,

e) 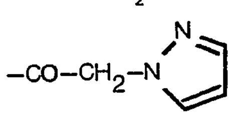 : Pyrazolyl-acetylderivate (und ihre Salze und Metallkomplexe)

f) 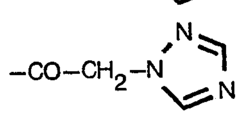 : Triazolyl-acetylderivate (und ihre Salze und Metallkomplexe)

g) gegebenenfalls halogenierte Furanoyl- und Tetrahydrofuranolyl-Derivate,

h) $\beta$-Alkoxy-propionyl-Derivate.

Es wurde überraschend gefunden, dass Verbindungen mit der Struktur der Formel I ein für die praktischen Bedürfnisse sehr günstiges Mikrobizid-Spektrum zum Schutze von Kulturpflanzen aufweisen. Kulturpflanzen seien im Rahmen Erfindung beispielsweise Getreide, Mais, Reis, Gemüse, Zuc-

kerrüben, Soja, Erdnüsse, Obstbäume, Zierpflanzen, vor allem aber Reben, Hopfen, Gurkengewächse (Gurken Kürbis, Melonen), Solanaceen wie Kartoffeln, Tabak und tomaten, sowie auch Bananen- Kakao- und Naturkautschuk-Gewächse.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Pilze eingedämmt oder vernichtet werden, wobei auch später zuwachsende Pflanzenteile von derartigen Pilzen verschont bleiben. Die Wirkstoffe sind gegen die den folgenden Klassen angehörenden phytopathogenen Pilze wirksam: Ascomycetes (z.B. Erysiphaceae, Sclerotinia, Helminthosporium): Basidiomycetes wie vor allem Rostpilze, Rhizoctonia; Fungi imperfecti (z.B. Moniliales, Piricularia); dann aber besonders gegen die der Klasse der Phycomycetes angehörenden Oomycetes wie Phytophthora, Peronospora, Pseudoperonospora, Pythium oder Plasmopara. Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft somit ferner die Verwendung der Verbindungen der Formel I zur Bekämpfung phytopathogener Mikroorganismen.

Die nachfolgenden Substituententypen und ihre Kombinationen untereinander werden bevorzugt:

$R_2$ = Wasserstoff oder Methyl,

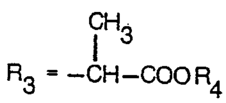 oder 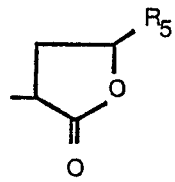 ,

$R_4$ = gegebenenfalls durch $C_1$—$C_2$-Alkoxy substituiertes $C_1$—$C_3$-Alkyl oder aber $C_2$—$C_4$-Alkenyl oder $C_3$—$C_4$-Alkinyl,

$R_5$ = Wasserstoff oder Methyl,

$R_6$ = 2-Furyl oder 2-Tetrahydrofuryl oder —$CH_2Z$ mit einer Bedeutung für Z von

a) $XR_7$ (X = Sauerstoff oder Schwefel)

b) —NH—$N(R_8)(R_9)$

c) —$OSO_2R_{10}$

d) 1,2,4-Triazolyl (1) und dessen Salze und Metallkomplexe, wobei $R_7$ im Falle von $R_3$ = Lacton entweder Allyl oder Propargyl bedeutet, und $R_7$ im Falle von $R_3$ = —$CH(CH_3)COOR_4$ Allyl, Propargyl oder $C_1$—$C_3$ Alkyl bedeutet, $R_8$ und $R_9$ unabhängig $C_1$—$C_2$ Alkyl bedeuten und $R_{10}$ $C_1$—$C_2$-Alkyl oder Monomethylamin darstellt.

Besonders bevorzugt sind solche Verbindungen der Formel I, bei denen $R_2$ = Wasserstoff oder $CH_3$,

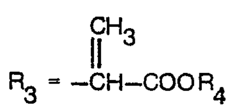 oder 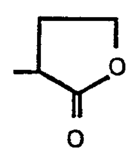

bedeuten, wobei $R_4$ = $C_1$—$C_3$ Alkyl, vorzugsweise Methyl darstellt, $R_6$ = 2-Furyl oder 2-Tetrahydrofuryl oder —$CH_2Z$ bedeutet, wobei Z im Falle $R_3$ = —$CH(CH_3)COOR_4$

a) —$OR_7$ mit $R_7$ = $C_1$—$C_3$ bedeutet,

b) —$OSO_2R_{10}$ bedeutet, wobei $R_{10}$ = $C_1$—$C_2$-Alkyl oder Monomethylamin ist, oder

c) 1,2,4-Triazolyl (1) darstellt, und wobei Z im Falle $R_3$ = Lacton eine der beiden Bedeutungen b) oder c) hat.

In der Gruppe der ungesättigten aliphatischen Acylverbindungen sind folgende Vertreter als Fungizide besonders bevorzugt:

A—1) N-(2-Propin-1-yloxyacetyl)-N-(2-methylnaphthyl)-N-(2-oxo-tetrahydrofuran-3-yl)-amin [=Verb. 2.5]

A—2) N-(2-Propen-1-yloxyacetyl)-N-(2-methylnaphthyl)-N-(2-oxo-tetrahydrofuran-3-yl)-amin [=Verb. 2.11]

In der Gruppe der gesättigten aliphatischen Acylverbindungen sind folgende Vertreter als Fungizide besonders bevorzugt:

B—1) N-(2-Methylnaphthyl)-N-methoxyacetyl-alanin-2-methoxyäthylester [=Verb. 1.20]

B—2) N-(2-Methylnaphthyl)-N-(isopropoxyacetyl)-alaninmethylester [=Verb. 1.21]

B—3 N-(2-Methylnaphthyl)-N-methoxyacetyl-alanin-2-isopropylester [=Verb. 1.60]

B—4 N-(2,3-Dimethylnaphthyl)-N-methoxyacetyl-alaninmethylester [=Verb. 1.67]

B—5 N-(2,3-Dimethylnaphthyl)-N-(3-methoxypropionyl)-alaninmethylester [=Verb. 1.69]

In der Gruppe der sulfonylierten und sulfamoylierten Acylverbindungen sind folgende Vertreter als Fungizide besonders bevorzugt:

C—1) N-(2-Methylnaphthyl)-N-methylsulfonyloxyacetyl-alaninmethylester [=Verb. 1.38]

C—2) N-(2-Methylnaphthyl)-N-(N'-methylsulfamoyloxyacetyl)-alaninmethylester [=Verb. 1.40]

C—3) N-(2-Methylnaphthyl)-N-methylsulfonyloxyacetyl-alaninisopropylester [=Verb. 1.56]

C—4) N-(2,3-Dimethylnaphthyl)-N-(N'-methysulfamoyloxyacetyl)-alaninmethylester [=Verb. 1.72]

C—5) N-Methylsulfonyloxyacetyl-N-(2-methylnaphthyl)-N-(2-oxo-tetrahydrofuran-3-yl)-amin [=Verb. 2.15]

C—6) N-(N'-Methylsulfamoyloxyacetyl)-N-(2-methylnaphthyl)-N-(2-oxo-tetrahydrofuran-3-yl)-amin [=Verb. 2.18]

C—7) N-(2,3-Dimethylnaphthyl)-N-(N'-methylsulfamoyloxyacetyl)-N-(2-oxo-tetrahydrofuran-3-yl)-amin [=Verb. 2.26]

C—8) N-(2,3-Dimethylnaphthyl)-N-(methylsulfonyloxyacetyl)-N-(2-oxo-tetrahydrofuran-3-yl)-amin [=Verb. 2.25]

In der Gruppe der Triazolylacetylverbindungen sind folgende Vertreter als Fungizide besonders bevorzugt:

D—1) N-(2-Methylnaphthyl)-N-[1,2,4-triazolyl(1)-acetyl]-alaninmethylester [=Verb. 1.10]

D—2) N-(2-Methylnaphthyl)-N-[1,2,4-triazolyl(1)-acetyl]-alanin-2-methoxyäthylester [=Verb. 1.29]

D—3) N-(2-Methylnaphthyl)-N-[1,2,4-triazolyl(1)-acetyl]-alaniniso-propylester [=Verb. 1.62]

D—4) N-(2,3-Dimethylnaphthyl)-N-[1,2,4-triazolyl(1)-acetyl]-alaninmethylester [=Verb. 1.68]

D—5) N-(2-Methylnaphthyl)-N-(2-oxo-tetrahydrofuran-3-yl)-N-[1,2,4-triazolyl(1)-acetyl]-amin [=Verb. 2.8]

D—6) N-(2-Methylnaphthyl)-N-(2-oxo-5-methyl-tetrahydrofuran-3-yl)-N-[1,2,4-triazolyl(1)-acetyl]-amin [=Verb. 2.14]

D—7) N-(2,3-Dimethylnaphthyl)-N-(2-oxo-tetrahydrofuran-3-yl)-N-[1,2,4-triazolyl(1)-acetyl]-amin [=Verb. 2.22]

In der Gruppe der Furanoyl- und der Tetrahydrofuranoyl-Verbindungen sind folgende Vertreter als Fungizide besonders bevorzugt:

E—1) N-(2-Methylnaphthyl)-N-(2-oxo-tetrahydrofuran-3-yl)-N-[tetrahydrofuran-3-yl)-N-[tetrahydrofuranoyl(2)]-amin [=Verb. 2.6]

E—2) N-(2,3-Dimethylnaphthyl)-N-(2-oxo-tetrahydrofuran-3-yl)-N-[tetrahydrofuranoyl(2)]-amin [=Verb. 2.21]

E—3) N-(2-Methylnaphthyl)-N-[tetrahydrofuranoyl(2)]-alaninmethylester [=Verb. 1.5]

E—4) N-(2-Methylnaphthyl)-N-[tetrahydrofuranoyl(2)]-alanin-2-methoxyäthylester [=Verb. 1.45]

E—5) N-(2-Methylnaphthyl)-N-[furanoyl(2)]-alanin-2-methoxyäthylester [=Verb. 1.37]

E—6) N-(2-Methylnaphthyl)-N-[tetrahydrofuranoyl(2)]-alaninisopropylester [=Verb. 1.64]

E—7) N-(2,3-Dimethylnaphthyl)-N-[furanoyl(2)]-alaninmethylester [=Verb. 1.65]

E—8) N-(2,3-Dimethylnaphthyl)-N-tetrahydrofuranoyl(2)]-alaninmethylester [=Verb. 1.66]

In der Gruppe der Hydrazinoacetyl-Verbindungen sind folgende Vertreter als Fungizide besonders bevorzugt:

F—1) N-(2-Methylnaphthyl)-N-(N'-2-phenylhydrazinoacetyl)-alaninmethylester [=Verb. 1.32]

F—2) N-(2-Methylnaphthyl)-N-(N',N'-2-dimethylhydrazinoacetyl)-alaninmethylester [=Verb. 1.33]

F—3) N-(2,3-Dimethylnaphthyl)-N-(N',N'-2-dimethylhydrazinoacetyl)-alaninmethylester [=Verb. 1.70]

Die nachfolgenden Beispiele dienen zur näheren Erläuterung der Erfindung, ohne dieselbe einzuschränken. die Temperaturen sind in Celsiusgraden angegeben. Prozente und Teile beziehen sich auf Gewicht. Sofern nicht anders vermerkt, ist bei der Nennung eines Wirkstoffs der Formel I stets das Isomeren-Gemisch gemeint.

Herstellungsbeispiele

Beispiel 1: Herstellung von

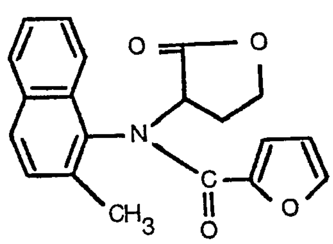

N-(2-Methylnaphthyl)-N-(2-oxo-tetrahydrofuran-3-yl)-N-(2-furanoyl)-amin [=Verb. 2.1]

a) 78,5 g 1-Amino-2-methylnaphthalin, 90,8 g 2-Brom-4-butyrolacton und 53 g Soda wurden in 250 ml Dimethylformamid 40 Stunden bei 70°C gerührt, abgekühlt, filtriert und das Lösungsmittel im Vakuum abgekampft. Der ölige Rückstand wurde in 1000 ml Methylenchlorid aufgenommen, dreimal mit je 200 ml Wasser gewaschen, über Natriumsulfat getrocknet, filtriert und das Methylenchlorid abgedampft. Der Rückstand wurde in Diäthyläther aufgenommen, mit Aktivkohle behandelt, filtriert und durch Abkühlen zur Kristallisation gebracht.

Der abfiltriert Niederschalg wurde durch Umkristallisieren aus Methanol gereinigt:

3-[N-(2-methylnaphthyl)]-amino-tetrahydro-2-furanon, Smp. 89—91°C.

b) 12 g des gemäss a) erhaltenen Zwischenproduktes, 13,7 g Furan-2-carbonsäurechlorid und 5,8 g Soda wurden 24 Stunden bei 60° gerührt, abgekühlt, filtriert und das Filtrat mit 200 ml Sodalösung und zweimal mit je 200 ml Waser gewaschen, über Natriumsulfat getrocknet, filtriert und das Lösungsmittel abgedampft.

Nach dem Umkristallisieren aus Essigsäureäthylester/Petrol-äther schmelzen die Kristalle des Diastereomerengemisches bei 141—166°.

Beispiel 2: Herstellung von

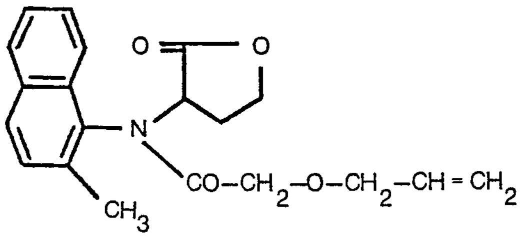

N-(2-Methylnaphthyl)-N-(2-oxo-tetrahydrofuran-3-yl)-n-allyloxyacetyl-amin [=Verb. 2.11]

14,5 g 3-[N-2-Methylnaphthyl(1)-amino]-butyrolacton und 9,3 g Allyloxyacetylchlorid werden unter $N_2$-Atmosphäre in 100 ml Toluol (absolut) 4 Std. rückfliessend erhitzt. Nach Beendigung der HCl-Entwicklung wird das Lösungsmittel verdampft. Das zurückbleibende braune Oel wird in 200 ml Toluol aufgenommen, mit Aktivkohle behandelt und filtriert. Nach dem Einengen der Lösung bleibt das Diastereomerengemisch der Verb. 2.11 als halbfeste Masse zurück,

$n_D^{22}$ 1,5911.

Beispiel 3: Herstellung von

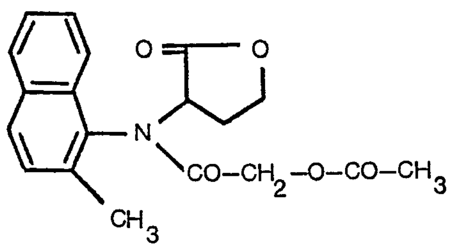

N-(2-Methylnaphthyl)-N-(2-oxo-tetrahydrofuran-3-yl)-N-acetoxyacetyl-amin [=Verb. 2.19]

Ein Gemisch von 18,2 g N-(2-Methylnaphthyl)-N-(2-oxo-tetrahydrofuran-3-yl)-N-chloracetylamin, 11,5 g wasserfreies Na-Acetat und 0,1 g KJ wird 16 Std. in 70 ml dimethylformamid (DMF) auf 110°C erhitzt. Danach wird das Reaktionsgemisch auf Raumtemperatur gekühlt, in 200 ml Wasser gegossen und dreimal mit 50 ml Aethylacetat extrahiert. Die vereinigten Extrakte werden mit 50 ml Wasser gewaschen, über Na-Sulfat getrocknet, filtriert und eingeengt. Das verbleibende dunkelbraune Oel wird über Kieselgel 60 (Korngrösse 0,06—0,2 mm) mit Hilfe von Chloroform/Diäthyläther (1:1) als Laufmittel gereinigt. Die letzten 10 von 12 Fraktionen werden vereinigt und das Laufmittel wird verdampft. Das zurückbleibende Diastereomerengemisch der Verbindung Nr. 2.19 erstarrt beim Abkühlen.

Beispiel 4:

a) Herstellung von N-(2-Methylnaphthyl)-N-(2-oxo-tetrahydrofuran-3-yl)-N-hydroxyacetylamin (Zwischenprodukt)

16,3 g der im Beispiel 3 hergestellten Verbindung Nr. 2.19 werden bei Raumtemperatur in 100 ml Methanol (absolut) gelöst und mit 1 g 25% ig. Na-Methylat versetzt, woraufhin das gewünschte Zwischenprodukt nach 5 Min. auszufallen beginnt. nach 2 Std. Rühren filtriert man das erhaltene braune Kristallpulver ab und wäscht mit Methanol, Smp. 191—195°C.

b) Herstellung von

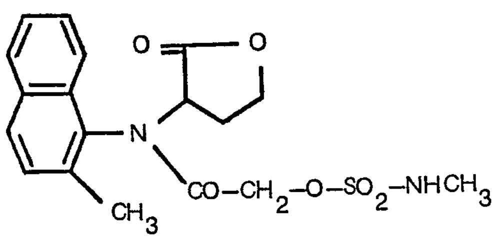

N-(2-Methylnaphthyl)-N-(2-oxo-tetrahydrofuran-3-yl)-N-(N'-methylsulfamoyloxyacetyl)-amin [=Verb. 2.18]

Zu 15 g des unter a) hergestellten Zwischenproduktes im Gemisch mit 4,3 g Pyridin und 80 ml Acetonitril (absolut) werden bei Raumtemperatur 7,1 g Methylamin-N-sulfonsäurechlorid, gelöst in 20 ml Acetonitrile (absolut), zugetropft, wobei sich das Reaktionsgemisch auf 35°C erwärmt. Nach 12 Std. Rühren bei Raumtemperatur werden nochmals 3,2 g Pyridin und 5,2 g Methylamin-N-sulfonsäurechlorid zugegeben. Nach 2 Std. Rühren wird das Reaktionsgemisch eingeengt, der Rückstand in 200 ml Aethylacetat aufgenommen, mit 50 ml Wasser gewaschen, über Na-sulfat getrocknet und filtriert. Nach dem Verdampfen des Lösungsmittels wird das verbleibende Oel mit Diäthyläther zur Kristallisation gebracht. Das kristalline Diastereomeren-Endprodukt wird in Gegenwart von Aktivkohle aus 500 ml Aethanol umkristallisiert; Smp. 190—194°C.

Beispiel 5:

Herstellung von

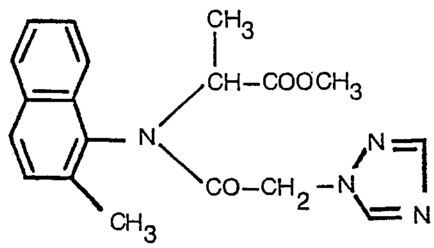

N-(2-Methylnaphthyl)-N-[1,2,4-triazolyl(1)-acetyl]-alaninmethylester [=Verb. 1.10]

15,9 g N-(2-Methylnaphthyl)-N-chloracetylalaninmethylester (hergestellt durch Reaktion von 2-Methylnaphthyl-1-amin und $\alpha$-Brompropionmethylester und Weiterreaktion des Zwischenprodukts mit Chloracetylchlorid), 9,1 g Na-Salz des 1,2,4-Triazols und 1 g KJ werden in 150 ml Methyläthylketon (absolut) 17 Std. rückfliessend erhitzt. Das Reaktionsgemisch wird auf Raumtemperatur gekühlt, filtriert und eingeengt. Der Rückstand wird in 200 ml Aethylacetat aufgenommen, die Lösung zweimal mit 50 ml Wasser gewaschen, mit Na-Sulfat getrocknet und eingeengt. Das verbleibende braune Oel wird über Kieselgel-60 mit Aceton als Laufmittel gereinigt. Die letzten 4 von 6 Fraktionen werden vereinigt und das Aceton abgedampft. Das Diastereomerengemisch des Endproduktes verbleibt als bräunliches Oel, $n_D^{23}$ 1.5920.

Beispiel 6:

Herstellung von

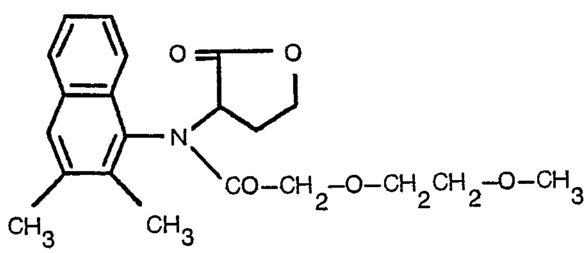

N-(2,3-Dimethylnaphthyl)-N-(2-oxo-tetrahydrofuran-3-yl)-N-(2-methoxy-äthoxy-acetyl)-amin [=Verb. 2.32]

Zu 15,3 g N-(2,3-Dimethylnaphthyl)-N-(2-oxo-tetrahydrofuran-3-yl)-amin in 120 ml Toluol werden 12,2 g 2-Methoxyäthoxy-acetylchlorid (MAC) in 20 ml Toluol und danach 8,1 g Triäthylamin in 20 ml Toluol getropft. Die auf 35°C ansteigende Reaktionswärme wird 2 Std. auf 40°C gesteigert. Dann werden weitere 2 g MAC und 1,5 g Triäthylamin zugetropft. Die Temperatur wird über Nacht bei 40°C gehalten. Danach werden nochmals 2 g MAC zugetropft. Nach 4 Std. wird das Reaktionsgemisch auf Raumtemperatur gekühlt und filtriert. Das Filtrat wird eingeengt. Man erhält einen harzigen Rückstand, der über Kieselgel-60 mit Aethylacetat als Laufmittel gereinigt wird. Das aus dem Rückstand anfallende feste Produkt wird aus Iso-propanol unter Zusatz von Aktivkohle umkristallisiert. Man erhält das farblose Diastereomerengemisch der Verbindung Nr. 2.32, Smp. 142—146°C.

Auf analoge Weise oder nach einer der hierin beschriebenen Methoden können folgende Verbindungen der Formel I hergestellt werden.

TABELLE I

$$(R = -\overset{CH_3}{\overset{|}{C}}HCOOR_4) \quad (R_2{=}H)$$

| Verb. No. | $R_6$ | $R_4$ | Physikal. Konstante, °C |
|---|---|---|---|
| 1.1 | 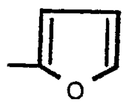 | $-CH_3$ | Smp. 128–130° |
| 1.2 | 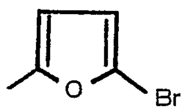 | $-CH_3$ | |
| 1.3 | $-CH_2OCH_3$ | $-CH_3$ | Sdp. 177–180°/ 0,1 mbar |
| 1.4 | $-CH_2OC_2H_5$ | $-CH_2-CH{=}CH_2$ | |
| 1.5 | 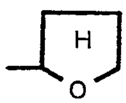 | $-CH_3$ | Smp. 101–106° |
| 1.6 | $-CH_2OC_2H_5$ | $-CH_2C{\equiv}CH$ | |
| 1.7 | $-CH_2CH_2OCH_3$ | $-CH_3$ | $n_D^{23}$ 1.5673 |
| 1.8 | $-CH_2OCH_3$ | $-CH_2-CH{=}CH_2$ | Oel |
| 1.9 | $-CH_2OCH_3$ | 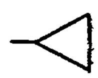 | Oel |
| 1.10 | 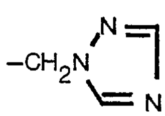 | $-CH_3$ | $n_D^{23}$ 1.5920 |
| 1.11 | 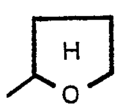 | $-CH_2-CH{=}CH_2$ | |
| 1.12 | 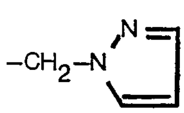 | $-CH_3$ | Oel |
| 1,13 | $-CH_2O-CH_2CH{=}CH_2$ | $-C_3H_7$-i | $n_D^{23}$ 1.5571 |
| 1.14 | $-CH_2OC_2H_4-OC_2H_5$ | $-CH_3$ | $n_D^{21}$ 1.5562 |

TABELLE I (Fortsetzung)

| Verb. No. | $R_6$ | $R_4$ | physikalische Konstante, °C |
|---|---|---|---|
| 1.15 | 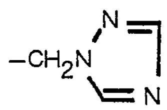 . 1/2 $CuCl_2$ | $-CH_3$ | Smp. >280° |
| 1.16 | $-CH_2OC_2H_5$ | $-CH_2CH_2OCH_3$ | |
| 1.17 | 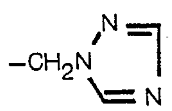 . 1/2 $H_2SO_4$ | $-CH_3$ | Smp. >280° |
| 1.18 | $-CH_2OCH_3$ | $-CH_2-C\equiv CH$ | |
| 1.19 | $-CH_2OC_2H_5$ | $-CH_3$ | |
| 1.20 | $-CH_2OCH_3$ | $-CH_2CH_2OCH_3$ | Oel |
| 1.21 | $-CH_2OC_3H_7$-i | $-CH_3$ | Oel |
| 1.22 | $-CH_2OCH_2-CH=CH_2$ | $-CH_3$ | $n_D^{23}$ 1.5700 |
| 1.23 | $-CH_2OCH_3$ | $-CH=CH_2$ | |
| 1.24 | $-CH_2OCH_3$ | $-C_4H_9$ sek. | |
| 1.25 | $-CH_2OCH_2C\equiv CH$ | $-CH_3$ | $n_D^{23}$ 1.5764 |
| 1.26 | $-CH_2SCH_3$ | $-CH_3$ | Oel |
| 1.27 | 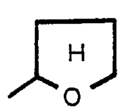 | $-CH_2CH_2Cl$ | Oel |
| 1.28 | $-CH_2SC_2H_5$ | $-CH_3$ | Oel |
| 1.29 | 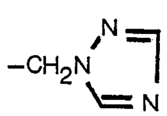 | $-CH_2CH_2OCH_3$ | Oel |

TABELLE I (Fortsetzung)

| Verb. No. | $R_6$ | $R_4$ | physikalische Konstante, °C |
|---|---|---|---|
| 1.30 | $-CH_2OCH(CH_3)C_2H_5)$ | $-CH_3$ | $n_D^{23}$ 1.5532 |
| 1.31 | $-CH_2OCH_3$ | $-CH_2CH_2Cl$ | |
| 1.32 | $-CH_2-NH-NH-$ 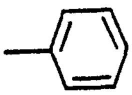 | $-CH_3$ | Oel |
| 1.33 | $-CH_2-NH-N(CH_3)_2$ | $CH_3$ | Oel |
| 1.34 | $-CH_2OCH_2C\equiv CH$ | $-C_3H_7$-i | $n_D^{23}$ 1.5615 |
| 1.35 | $-CH_2OC_2H_5$ | $-CH_2CH_2Cl$ | |
| 1.36 | $-CH_2-OSO_2N(CH_3)_2$ | $-CH_3$ | |
| 1.37 | 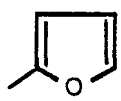 | $-CH_2CH_2OCH_3$ | Smp. 136–143° |
| 1.38 | $-CH_2-OSO_2CH_3$ | $-CH_3$ | Smp. 158–166° |
| 1.39 | $-CH_2$ 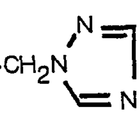 | $-C_3H_7$-n | Oel |
| 1.40 | $-CH_2-OSO_2NHCH_3$ | $-CH_3$ | Smp. 141–153° |
| 1.41 | $-CH_2-O-COCH_3$ | $-C_3H_7$-i | Oel |
| 1.42 | $-CH_2-O\overset{O}{\overset{\|}{C}}CH_3$ | $-CH_3$ | $n_D^{23}$ 1.5676 |
| 1.43 | $-CH_2-O\overset{O}{\overset{\|}{C}}CH_2OCH_3$ | $-CH_3$ | Oel |
| 1.44 | $-CH_2-CH_2OCH_3$ | $-C_3H_7$-i | Oel |

TABELLE I (Fortsetzung)

| Verb. Nr. | $R_6$ | $R_4$ | physikalische Konstante, °C |
|---|---|---|---|
| 1.45 | 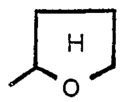 | $-CH_2CH_2OCH_3$ | Smp. 108–114° |
| 1.46 | $-CH_2O-SO_2NHC_2H_5$ | $CH_3$ | Smp. 130–149° |
| 1.47 | $-CH_2OCH_3$ | $-C_3H_7$-n | |
| 1.48 | $-CH_2OCH_3$ | 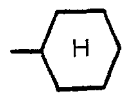 | |
| 1.49 | 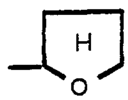 | $-C_2H_5$ | Smp. 96–105° |
| 1.50 | $-CH_2OC_2H_5$ | $-CH=CH_2$ | |
| 1.51 | 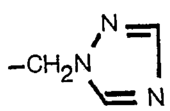 | $-C_2H_5$ | Oel |
| 1.52 | 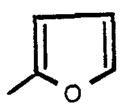 | $-C_3H_7$-n | |
| 1.53 | $-CH_2OC_2H_4OCH_3$ | $-CH_3$ | $n_D^{23}$ 1.5603 |
| 1.54 | $-CH_2OCH_3$ | $-C_2H_5$ | |
| 1.55 | $-CH_2OCH(CH_3)C_2H_5$ | $-C_3H_7$-i | $n_D^{22}$ 1.5435 |
| 1.56 | $-CH_2-OSO_2CH_3$ | $-C_3H_7$-i | Smp. 160–168° |
| 1.57 | $-CH_2NHN(CH_3)_2$ | $-C_3H_7$-i | Oel |
| 1.58 | $-CH_2OC_2H_5$ | $-C_3H_7$-i | Sdp. 188–190°/ 0.1 mbar |
| 1.59 | $-CH_2O-C_2H_4-OCH_3$ | $-C_3H_7$-i | $n_D^{22,5}$ 1.5512 |

TABELLE I (Fortsetzung)

| Verb. No. | $R_6$ | $R_4$ | physikalische Konstante, °C |
|---|---|---|---|
| 1.60 | $-CH_2OCH_3$ | $-C_3H_7$-i | Smp. 102–115° |
| 1.61 | $-CH_2NHNH-C_6H_5$ | $-C_3H_7$-i | Oel |
| 1.62 | 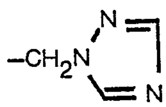 | $-C_3H_7$-i | Oel |
| 1.63 | 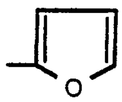 | $-C_3H_7$-i | $n_D^{23}$ 1.5901 |
| 1.64 | 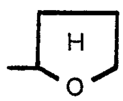 | $-C_3H_7$-i | $n_D^{23}$ 1.5665 |

Auf analoge Weise können auch folgende Verbindungen der Formel I hergestellt werden:

TABELLE I (Fortsetzung)

($R_3$=–CH($CH_3$)$COOCH_3$) ($R_2$=$CH_3$)

| Verb. No. | $R_6$ | physikalische Konstante, °C |
|---|---|---|
| 1.65 | 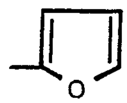 | Smp. 133–135° |
| 1.66 | 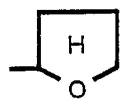 | Oel |
| 1.67 | $-CH_2OCH_3$ | Smp. 92–100° |
| 1.68 | 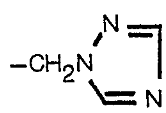 | Oel |
| 1.69 | $-CH_2CH_2OCH_3$ | Smp. 98–108° |
| 1.70 | $-CH_2-NH-N(CH_3)_2$ | Oel |
| 1.71 | $-CH_2-O-SO_2CH_3$ | halbfest |
| 1.72 | $-CH_2-OSO_2NHCH_3$ | Smp. 162–170° |
| 1.73 | $-CH_2-O-CO-CH_3$ | |
| 1.74 | $-CH_2-O-CH_2-C{\equiv}CH$ | Oel |
| 1.75 | $-CH_2-O-C_2H_5$ | Oel |

Auf analoge Weise oder nach einer der hierin beschriebenen Methoden können folgende Verbindungen der Formel I hergestellt werden:

TABELLE 2

($R_3$ = 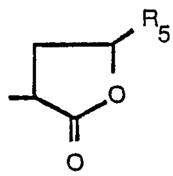 )

| Verb. No. | $R_2$ | $R_6$ | $R_5$ | physikalische Konstante, °C |
|---|---|---|---|---|
| 2.1 | H | 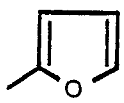 | H | Smp. 141–166° |
| 2.2 | H | 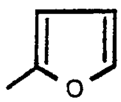 | $CH_3$ | Smp. 138–149° |
| 2.3 | H | 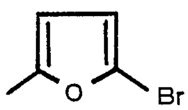 | H | Smp. 165–210° |
| 2.4 | H | $-CH_2NH-NH-$ 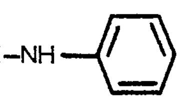 | H | Oel |
| 2.5 | H | $-CH_2OCH_2C{\equiv}CH$ | H | $n_D^{23,5}$ : 1,5971 |
| 2.6 | H | 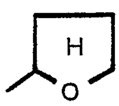 | H | Smp. 179–205° |
| 2.7 | H | $-CH_2CH_2OCH_3$ | H | Oel |
| 2.8 | H | $-CH_2$ 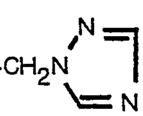 | H | Smp. 75–100° |
| 2.9 | H | $-CH_2-NHN(CH_3)_2$ | H | Oel |
| 2.10 | H | $-CH_2-$ 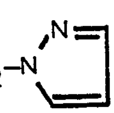 | H | Oel |

TABELLE 2 (Fortsetzung)

| Verb. No. | $R_2$ | $R_6$ | $R_5$ | physikalische Konstante, °C |
|---|---|---|---|---|
| 2.11 | H | $-CH_2OCH_2CH{=}CH_2$ | H | $n_D^{22}$ : 1,5911 |
| 2.12 | H | $-CH_2-OSO_2N(CH_3)_2$ | H | Smp. 133–170° |
| 2.13 | H | 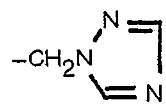 . 1/2 $H_2SO_4$ | H | Smp. >280° |
| 2.14 | H | 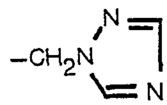 | $CH_3$ | Smp. 68–86° |
| 2.15 | H | $-CH_2-OSO_2CH$ | H | Smp. 182–191° |
| 2.16 | H | $-CH_2-NHN(CH_3)_2$ | $CH_3$ | braunes Oel |
| 2.17 | H | 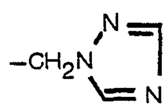 1/2 $CuCl_2$ | H | Smp. >300° |
| 2.18 | H | $-CH_2-OSO_2NHCH_3$ | H | Smp. 190–194° |
| 2.19 | H | 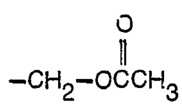 | H | Smp. 50–70° |
| 2.20 | $CH_3$ | 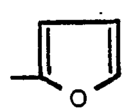 | H | Smp. 197–199° |
| 2.21 | $CH_3$ | 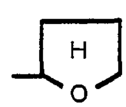 | H | Smp. 230–234° |
| 2.22 | $CH_3$ | 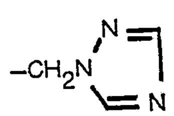 | H | Diast. I Smp. 209–210°<br>Diast. II Smp. 219–221°<br>Diast. Gem. Smp. 79–188° |

TABELLE 2 (Fortsetzung)

| Verb. No. | $R_2$ | $R_6$ | $R_5$ | physikalische Konstante, °C |
|---|---|---|---|---|
| 2.23 | $CH_3$ | $-CH_2CH_2OCH_3$ | H | Oel |
| 2.24 | $CH_3$ | $-CH_2-NH-N(CH_3)_2$ | H | Oel |
| 2.25 | $CH_3$ | $-CH_2-OSO_2CH_3$ | H | Smp. 182–205° |
| 2.26 | $CH_3$ | $-CH_2-OSO_2NHCH_3$ | H | Smp. 144–146° |
| 2.27 | $CH_3$ | $-CH_2-O\overset{O}{\overset{\|}{C}}CH_3$ | H | Smp. 171–172° |
| 2.28 | H | $-CH_2-O-C_2H_4-OCH_3$ | H | $n_D^{22}$ 1,5832 |
| 2.29 | H | $-CH_2O-SO_2NHC_2H_5$ | H | Smp. 143–145° |
| 2.30 | H | $-CH_2-O-C_2H_4-OC_2H_5$ | H | $n_D^{21,5}$ 1,5750 |
| 2.31 | $CH_3$ | $-CH_2-O-CH_2-C \equiv CH$ | H | Smp. 148–150° |
| 2.32 | $CH_3$ | $-CH_2-O-C_2H_4-O-CH_3$ | H | Smp. 142–146° |
| 2.33 | $CH_3$ | 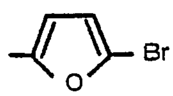 | H | Smp. 201–209° |
| 2.34 | $CH_3$ | $-CH_2-O-CH_2-CH=CH_2$ | H | Smp. 92–99° |
| 2.35 | $CH_3$ | $-CH_2-O-C_2H_4-OC_2H_5$ | H | Smp. 124–126° |
| 2.36 | $CH_3$ | $-CH_2-O-SO_2NHC_2H_5$ | H | Smp. 130–132° |
| 2.37 | $CH_3$ | $-CH_2-OSO_2N(CH_3)_2$ | H | Smp. 145–162° |

## Formulierungsbeispiele

Beispiel 2 Stäubemittel:

Zur Herstellung eines a) 5%igen und b) 2%igen Stäubemittels werden die folgenden Stoffe verwendet:

a) 5 Teile Wirkstoff
95 Teile Talkum;

b) 2 Teile Wirkstoff
1 Teil hochdisperse Kieselsäure,
97 Teile Talkum:

Die Wirkstoffe werden mit den Trägerstoffen vermischt und vermahlen und können in dieser Form zur Anwendung verstäubt werden.

Beispiel 3 Granulat:

Zur Herstellung eines 5%igen Granulates werden die folgenden Stoffe verwendet:

5 Teile Wirkstoff
0,25 Teile Epichlorhydrin,
0,25 Teile Cetylpolyglykoläther,
3,50 Teil Polyäthylenglykol
91 Teile Kaolin (Korngrösse 0,3—0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 4 Spritzpulver:

Zur Herstellung eines a) 70%igen, b) 40%igen c), d) 25%igen und e) 10%igen Spritzpulvers werden folgende Bestandteile verwendet:

a) 70 Teile Wirkstoff
5 Teile Natriumdibutylnaphthylsulfonat,
3 Teile Naphthalinsulfonsäuren-Phenolsulfonsaüren-Formaldehyd-Kondensat 3:2:1,
10 Teile Kaolin
12 Teile Champagne-Kreide;

b) 40 Teile Wirkstoff
5 Teile Ligninsulfonsäure-Natriumsalz,
1 Teil Dibutylnaphthalinsulfonsäure-Natriumsalz,
54 Teile Kieselsäure;

c) 25 Teile Wirkstoff
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

d) 25 Teile Wirkstoff
2,5 Teile Isooctylphenoxy-polyoxyäthylen-äthanol,
1,7 Teile Champagne-Kreide/Hydroxyäthylcellulose-Gemisch (1:1),
8,3 Teile Natriumaluminiumsilikat,
16,5 Teile Kieselgur,
46 Teile Kaolin;

e) 10 Teile Wirkstoff
3 Teile Gemisch der Natriumsalze von gesättigten Fettalkohol-sulfaten,
5 Teile Naphthalinsulfonsäure-Formaldehyd-Kondensat,
82 Teile Kaolin;

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

Beispiel 5 Emulgierbare Konzentrate:

Zur Herstellung eines 25%igen emulgierbaren Konzentrates werden folgende Stoffe verwendet:

25 Teile Wirkstoff
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen der gewünschten Anwendungskonzentration hergestellt werden, die besonders zur Blattapplikation geeignet sind.

Biologische Beispiele

*1. Wirkung gegen Phytophthora infestans auf Tomatenpflanzen*

a) Residual-protektive Wirkung

Tomatenpflanzen wurden nach 3-wöchiger Anzucht mit einer aus Spritzpulver des Wirkstoffes hergestellten Spritzbrühe (0,02% oder 0,006% Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90—100% relativer Luftfeuchtigkeit und 20°C.

b) Residual-kurative Wirkung

Tomatenpflanzen wurden nach 3-wöchiger Anzucht mit einer Sporangiensuspension des Pilzes infiziert. Nach einer Inkubation von 22 Stunden in einer Feuchtkammer bei 90—100% relativer Luftfeuchtigkeit und 20°C wurden die infizierten Pflanzen getrocknet und mit einer aus Spritzpulver des Wirkstoffes hergestellten Spritzbrühe (0,02% oder 0,006% Aktivsubstanz) besprüht. Nach dem Antrocknen des Spritzbelages wurden die behandelten Pflanzen wieder in die Feuchtkammer gebracht. Die beurteilung des Pilzbefalls erfolgte 5 Tage nach der Infektion. Bei einer Anwendungskonzentration von 0,02% oder von 0,006% (*) im Test a) und b) senkten die folgenden Verbindungen den Pilzbefall auf 0—5%:

| | | | | | | |
|---|---|---|---|---|---|---|
| 1.1 | 1.26* | 1.44 | 1.63 | 2.3 | 2.17* | 2.32 |
| 1.3 | 1.27 | 1.45* | 1.64* | 2.4 | 2.18* | 2.33 |
| 1.5* | 1.28 | 1.46* | 1.65* | 2.5 | 2.20 | 2.34* |
| 1.7 | 1.29* | 1.51* | 1.66* | 2.6* | 2.21* | 2.35 |
| 1.10* | 1.31* | 1.53 | 1.67* | 2.7 | 2.22* | 2.36* |
| 1.12* | 1.32 | 1.54 | 1.68* | 2.8* | 2.23 | 2.37* |
| 1.15* | 1.33* | 1.55 | 1.69 | 2.9* | 2.24* | |
| 1.16* | 1.35 | 1.56* | 1.70* | 2.10 | 2.25 | |
| 1.17* | 1.36 | 1.57* | 1.71* | 2.11 | 2.26* | |
| 1.19* | 1.37 | 1.58* | 1.72* | 2.12 | 2.27 | |
| 1.20* | 1.38* | 1.59 | 1.74* | 2.13 | 2.28 | |
| 1.21 | 1.39 | 1.60* | 1.75* | 2.14* | 2.29* | |
| 1.22 | 1.40* | 1.61* | 2.1 | 2.15 | 2.30 | |
| 1.25* | 1.42 | 1.62* | 2.2 | 2.16* | 2.31* | |

c) Systemische Wirkung

Zu Tomatenpflanzen wurde nach 3-wöchiger Anzucht eine aus Spritzpulver des Wirkstoffes hergestellte Spritzbrühe gegossen (0,006% Aktivsubstanz bezogen auf das Erdvolumen). Es wurde darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90—100% relativer Luftfeuchtigkeit und 20°C. Sämtliche bei den Tests a) und b) genannten Wirkstoffe verhüteten infolge systemischer Wirkung einen Krankheitsbefall an den Pflanzen. Die Pflanzen zeigten ein gesundes Aussehen.

2. *Wirkung gegen Plasmopara viticola auf Reben* Im 4—5 Blatt-Stadium wurden Rebensämlinge mit einer aus Spritzpulver des Wirkstoffes hergestellten Spritzbrühe (0.02% Aktivsubstanz) besprüht. Nach 24 Stunden wurden die behandelten Pflanzen mit einer Sporangien-suspension des Pilzes infiziert. Nach einer Inkubation während 6 Tagen bei 95—100% relative Luftfeuchtigkeit und 20°C wurde der Pilzbefall beurteilt.

Die im biologischen Beispiel 1 genannten Wirkstoffe sowie die Wirkstoffe Nr. 1.49 und Nr. 2.19 verhüteten den Pilzbefall vollständig oder fast vollständig (0—5% Befall).

3. *Wirkung gegen Pythium debaryanum auf Rüben.* a) Wirkung nach *Bodenapplikation.* Der Pilz wurde auf karottenschnitzel-Nährlösung kultiviert und einer Erde-Sand-Mischung beigegeben. Die so infizierte Erde wurde in Blumentöpfe abgefüllt und mit Zuckerrübensamen besät. Gleich nach der Aussaat wurden die als Spritzpulver formulierten Versuchspräparate als wässrige Suspensionen über die Erde gegossen (20 ppm Wirkstoff bezogen auf das Ervolumen). Die Töpfe wurden darauf während 2—3 Wochen im Gewächshaus bei ca. 20°C aufgestellt. Die Erde wurde dabei durch vorsichtiges Ueberbrausen mit der Gieskanne gleichmässig feucht gehalten.

b) *Wirkung nach Beizapplikation.* Der Pilz wurde auf Karottenschnitzel-Nährlösung kultiviert und einer Erde-Sand-Mischung beigegeben. Die so infizierte Erde wurde in Erdschalen abgefüllt und mit Zuckerrübensamen besät, die mit den als Beizpulver formulierten Versuchspräparaten gebeizt worden waren (0,06% Wirkstoff). Die besäten Töpfe wurden während 2—3 Wochen im Gewächshaus bei ca. 20°C aufgestellt. Die Erde wurde dabei durch leichtes Ueberbrausen gleichmässig feucht gehalten.

Bei der Auswertung beider Versuche wurde die Prozentzahl an aufgelaufenen Zuckerrübenpflanzen sowie der Anteil gesunder und kranker Pflanzen bestimmt.

Sämtliche der in den vorstehenden biologischen Beispielen 1 und 2 aufgeführten Wirkstoffe zeigten volle Wirkung gegen Pythium spp. (über 90% aufgelaufene Pflanzen). Die Pflanzen hatten ein gesundes Aussehen.

4. *Wirkung gegen Cercospora arachidicola auf Erdnusspflanzen* 10—15 cm hohe Erdnusspflanzen wurden mit einer aus Spritzpulver der Wirksubstanz hergestellten Spritzbrühe (0,02% Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

Neben anderen erwiesen sich Verbindungen der Untergruppen a) = aliphatische Acylverbindungen, c) = Sulfonyl- und Sulfamoyl-acetylderivate, e) = Pyrazolyl-acetylderivate und f) = Triazolylacetylderivate als besonders stark wirksam gegen Cercospora-Befall. Mit folgenden Wirkstoffen wurde der Pilzbefall fast vollständig verhütet (0—10% Befall):

a) Nr. 1.20, 1.21, 1.25, 1.34, 1.60, 1.67, 1.69, 1.75, 2.5, 2.11, 2.31, 2.34;

b) Nr. 1.40, 1.46, 1.72, 2.18, 2.26, 2.29, 2.36;

e) Nr. 1,12, 2.10;

f) Nr. 1.10, 1.15, 1.17, 1.29, 1.62, 1.68, 2.8, 2.13, 2.14, 2.17, 2.22;

sowie mit den Verbindungen Nr. 1.70 und 2.21.

**Patentansprüche für die Vertragsstaaten: BE CH DE FR GB IT LI NL SE**

1. Acylierte Naphthylamine der Formel I

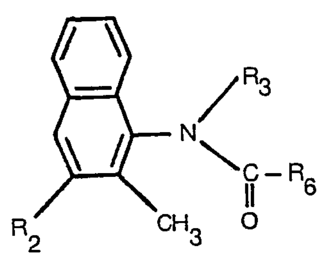

(I)

worin $R_2$ Wasserstoff oder Methyl bedeutet, $R_3$ für $—CH(CH_3)COOR_4$ oder

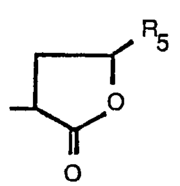

steht, wobei $R_4$ gegebenenfalls durch Halogen oder $C_1—C_2$-Alkoxy substituiertes $C_1—C_4$-Alkyl, $C_2—C_4$-Alkenyl, $C_3—C_4$-Alkinyl oder $C_3—C_7$-Cycloalkyl und $R_5$ Wasserstoff oder Methyl bedeuten und $R_6$ gegebenenfalls durch Halogen substituiertes 2-Furyl oder 2-Tetrahydrofuryl, $\beta$-($C_1—C_4$)-Alkoxyäthyl oder die Gruppe $CH_2Z$ bedeutet, wobei Z für eine der Gruppen a) $—X—R_7$, b) $—NH—N(R_8)(R_9)$, c) $—OSO_2R_{10}$, d) $—O(CO)R_{11}$, e) 1,2-Pyrazolyl (1) oder f) 1,2,4-Triazolyl (1) steht, unter Einschluss ihrer Salze und Metallkomplexe, und X Sauerstoff oder Schwefel ist, $R_7$ ein durch $C_1—C_2$-Alkoxy substituiertes $C_1—C_6$-Alkyl, oder aber $C_3—C_4$-Alkenyl oder $C_3—C_4$-Alkinyl bedeutet, $R_8$ Wasserstoff oder $C_1—C_3$-Alkyl, $R_9$ $C_1—C_3$-Alkyl oder Phenyl, $R_{10}$ $C_1—C_4$-Alkyl oder Mono- oder Di($C_1—C_3$)-Alkylamin und $R_{11}$ gegebenenfalls durch $C_1—C_2$-Alkoxy substituiertes $C_1—C_3$-Alkyl darstellen, wobei im Falle $R_3 = —CH(CH_3)COOR_4$ der Substituent $R_7$ auch $C_1—C_6$-Alkyl bedeuten kann.

2. Acylierte Naphthylamine der Formel I gemäss Anspruch 1, worin $R_2$ Wasserstoff oder Methyl ist,

$R_3 = -CH(CH_3)COOR_4$ oder 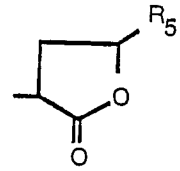

bedeutet,

$R_4$ gegebenenfalls durch $C_1$—$C_2$-Alkoxy substituiertes $C_1$—$C_3$-Alkyl oder aber $C_2$—$C_4$-Alkenyl oder $C_3$—$C_4$-Alkinyl darstellt,

$R_5$ Wasserstoff oder Methyl ist und

$R_6$ = 2-Furyl, 2-Tetrahydrofuryl oder $CH_2Z$ darstellt, mit einer Bedeutung für Z von

a) —X—$R_7$, worbei X Sauerstoff oder Schwefel ist,

b) —NH—$N(R_8)(R_9)$,

c) —$OSO_2R_{10}$,

d) 1,2,4-Triazolyl (1) und dessen Salze und Metallkomplexe, wobei

$R_7$ im Falle von $R_3$ = Lacton entweder Allyl oder Propargyl bedeutet, und

$R_7$ im Falle von $R_3$ = —$CH(CH_3)COOR_4$ wahlweise Allyl, Propargyl oder $C_1$—$C_3$-Alkyl bedeutet,

$R_8$ und $R_9$ unabhängig $C_1$—$C_2$-Alkyl bedeuten und

$R_{10}$ $C_1$—$C_2$-Alkyl oder Monomethylamin

darstellt.

3. Acylierte Naphthylamine der Formel I gemäss Anspruch 1, worin $R_2$ Wasserstoff oder Methyl ist,

$R_3 = -CH(CH_3)-COOR_4$ oder 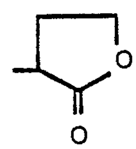

bedeutet, wobei

$R_4$ $C_1$—$C_3$-Alkyl darstellt,

$R_6$ = 2-Furyl, 2-Tetrahydrofuryl oder —$CH_2Z$ bedeutet, wobei Z im Falle von $R_3$ = —$CH(CH_3)COOR_4$ wahlweise

a) —$OR_7$ mit $R_7$ = $C_1$—$C_3$-Alkyl bedeutet, oder

b) —$OSO_2R_{10}$ bedeutet, wobei $R_{10}$ = $C_1$—$C_2$-Alkyl oder Monomethylamin ist, oder

c) 1,2,4-Triazolyl (1) darstellt, und wobei Z im Falle $R_3$ = Lacton eine der beiden vorstehenden Bedeutungen b) oder c) hat.

4. Eine Verbindung aus der Gruppe
N-(2-Propin-1-yloxyacetyl)-N-(2-methylnaphthyl)-N-(2-oxo-tetrahydrofuran-3-yl)-amin,
N-(2-Methylnaphthyl)-N-(2-propin-1-yl-oxyacetyl)-alaninmethylester oder
N-(2-Methylnaphthyl)-N-(2-propin-1-yl-oxyacetyl)-alaninisopropylester.

5. N-(2-Propen-1-yloxyacetyl)-N-(2-methylnaphthyl)-N-(2-oxo-tetrahydrofuran-3-yl)-amin.

6. Eine Verbindung aus der Gruppe
N-(2-Methylnaphthyl)-N-methoxyacetyl-alanin-2-methoxyäthylester,
N-(2-Methylnaphthyl)-N-methoxyacetyl-alanin-2-isopropylester,
N-(2,3-Dimethylnaphthyl)-N-methoxyacetyl-alaninmethylester.

7. N-(2-Methylnaphthyl)-N-(isopropoxyacetyl)-alaninmethylester.

8. N-(2,3-Dimethylnaphthyl)-N-äthoxyacetyl-alaninmethylester.

9. N-(2,3-Dimethylnaphthyl)-N-($\beta$-methoxy-äthoxyacetyl)-N-(2-oxotetrahydrofuran-3-yl)-amin.

10. Eine Verbindung aus der Gruppe
N-(2-Methylnapthyl)-N-methylsulfonyloxyacetyl-alaninmethylester,
N-(2-Methylnaphthyl)-N-methylsulfonyloxyacetyl-alaninisopropylester,
N-Methylsulfonyloxyacetyl-N-(2-methylnaphthyl)-N-(2-oxo-tetrahydrofuran-3-yl)-amin.
N-(2,3-Dimethylnaphthyl)-N-(methylsulfonyloxyacetyl)-N-(2-oxo-tetrahydrofuran-3-yl)-amin.

11. Eine Verbindung aus der Gruppe
N-(2-Methylnaphthyl)-N-(N'-methylsulfamoyloxyacetyl)-alanin-methylester,
N-(2,3-Dimethylnaphthyl)-N-(N'-methylsulfamoyloxyacetyl)-alanin-methyester,
N-(N'-Methylsulfamoyloxyacetyl)-N-(2-methylnaphthyl)-N-(2-oxo-tetrahydrofuran-3-yl)-amin,
N-(2,3-Dimethylnaphthyl)-N-(N'-methylsulfamoyloxyacetyl)-N-(2-oxo-tetrahydrofuran-3-yl)-amin.

12. Eine Verbindung aus der Gruppe
N-(2-Methylnaphthyl)-N-[1,2,4-triazolyl (1)-acetyl]-alaninmethylester,
N-(2-Methylnaphthyl)-N-[1,2,4-triazolyl (1)-acetyl]-alanin-2-methoxyäthylester,
N-(2-Methylnaphthyl)-N-[1,2,4-triazolyl (1)-acetyl]-alaninisopropylester,
N-(2,3-Dimethylnaphthyl)-N-[1,2,4-triazolyl (1)-acetyl]-alaninmethylester,
N-(2-Methylnaphthyl)-N-(2-oxo-tetrahydrofuran-3-yl)-N-[1,2,4-triazolyl-(1)-acetyl]-amin,
N-(2-Methylnaphthyl)-N-(2-oxo-5-methyl-tetrahydrofuran-3-yl)-N-[1,2,4-triazolyl (1)-acetyl]-amin,

N-(2,3-Dimethylnaphthyl)-N-(2-oxo-tetrahydrofuran-3-yl)-N-[1,2,4-triazolyl (1)-acetyl]-amin.

13. Eine Verbindung aus der Gruppe
N-(2-Methylnaphthyl)-N-(2-oxo-tetrahydrofuran-3-yl)-N-[tetrahydrofuran-3-yl)-N-[tetrahydrofuranoyl (2)]-amin,
N-(2,3-Dimethylnaphthyl)-N-(2-oxo-tetrahydrofuran-3-yl)-N-[tetrahydrofuranoyl (2)]-amin,
N-(2-Methylnaphthyl)-N-[tetrahydrofuranoyl (2)]-alaninmethylester,
N-(2-Methylnaphthyl)-N-[tetrahydrofuranoyl (2)]-alanin-3-methoxyäthylester,
N-(2-Methylnaphthyl)-N-[tetrahydrofuranoyl (2)]-alaninisopropylester,
N-(2,3-Dimethylnaphthyl)-N-[tetrahydrofuranoyl (2)]-alaninmethylester.

14. Eine Verbindung aus der Gruppe
N-(2,3-Dimethylnaphthyl)-N-[furanoyl (2)]-alaninmethylester,
N-(2-Methylnaphthyl)-N-[furanoyl (2)]-alanin-2-methoxyäthylester.

15. N-(2-Methylnaphthyl-N-(N'-2-phenylhydrazinoacetyl)-alaninmethylester.

16. Eine Verbindung aus der Gruppe
N-(2-Methylnaphthyl)-N-(N',N'-2-dimethylhydrazinoacetyl)-alaninmethylester,
N-(2,3-Dimethylnaphthyl-N-(N',N'-dimethylhydrazinoacetyl)-alaninmethylester.

17. Verfahren zur Herstellung von acylierten Naphthylaminen der Formel I gemäss Anspruch 1, dadurch gekennzeichnet, dass man

A. eine Verbindung der Formel II mit einer Carbonsäure der Formel III oder ihrem Säurehalogenid oder Säureanhydrid acyliert

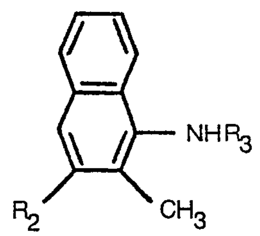

$HO-CO-R_6$ ,

(II) (III)

oder dass man

B. eine Verbindung der Formel IV oder ihr Salz mit wahlweise einer Verbindung der Formel V, VI oder VII zur Reaktion bringt,

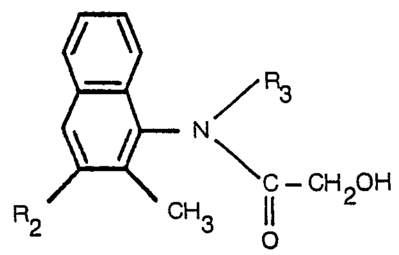

+

$Hal-SO_2R_{10}$ (V)

$Hal-CO-R_{11}$ (VI)

$Hal-R_7$ (VII)

(IV)

oder dass man

C. eine Verbindung der Formel X wahlweise mit einer Verbindung der Formel XI, XII, XIII oder XIIIa zur Reaktion bringt,

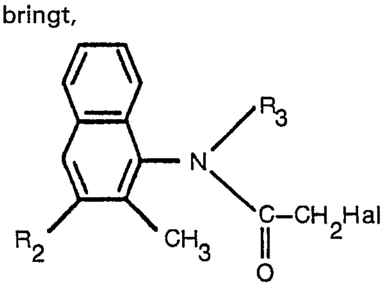

+

$MXR_7$ (XI)

$MO(CO)R_{11}$ (XII)

$NH_2N(R_8)(R_9)$ (XIII)

M–Azolyl (XIIIa)

(X)

wobei in den vorstehend genannten Formeln die Substituenten $R_2$, $R_3$, $R_6$ bis $R_{11}$ und X die für Formel I angegebenen Bedeutungen haben, während "Hal" für Halogen, "M" Wasserstoff oder ein Metallkation, bedeutet, und "Azolyl" ein 1,2-Pyrazolyl (1) oder 1,2,4-Triazolyl (1) darstellt.

18. Pflanzenfungizides Mittel enthaltend als Wirkstoff mindestens eine Verbindung der Formel I

gemäss Anspruch 1, zusammen mit einem geeigneten Trägerstoff und/oder einem oberflächenaktiven Zusatz.

19. Pflanzenfungizides Mittel gemäss Anspruch 18 enthaltend als Wirkstoff eine Verbindung der Formel I gemäss einem der Ansprüche 2 bis 16.

20. Verwendung einer Verbindung der Formel I des Anspruchs 1 zur Bekämpfung oder zur Verhütung von Pilzbefall an Pflanzen.

21. Verwendung eines Verbindung der Formel I des Anspruchs 1 gemäft einem der Ansprüche 2 bis 16 gemäft Anspruche 20.

**Patentansprüche für den Vertragsstaat: AT**

1. Pflanzenfungicides Mittel enthaltend mindestens ein N-acyliertes alpha-Naphthylamin der allgemeinen Formel I als Wirkstoff

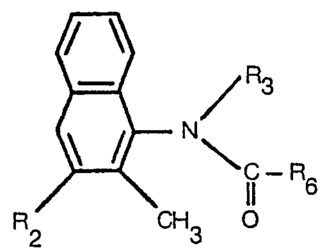

(I)

worin $R_2$ Wasserstoff oder Methyl bedeutet, $R_3$ für $—CH(CH_3)COOR_4$ oder

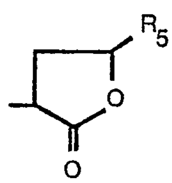

steht, wobei $R_4$ gegebenenfalls durch Halogen oder $C_1—C_2$-Alkoxy substituiertes $C_1—C_4$-Alkyl, $C_2—C_4$-Alkenyl, $C_3—C_4$-Alkinyl oder $C_3—C_7$-Cycloalkyl und $R_5$ Wasserstoff oder Methyl bedeuten und $R_6$ gegebenenfalls durch Halogen substituiertes 2-Furyl oder 2-Tetrahydrofuryl, $\beta$-($C_1—C_4$)-Alkoxyäthyl oder die Gruppe $CH_2Z$ bedeutet, wobei Z für eine der Gruppen a) $—X—R_7$, b) $—NH—N(R_8)(R_9)$, c) $—SOS_2R_{10}$, d) $—O(CO)R_{11}$, e) 1,2-Pyrazolyl (1) oder f) 1,2,4-Triazolyl (1) steht, unter Einschluss ihrer Salze und Metallkomplexe, und X Sauerstoff oder Schwefel ist, $R_7$ ein durch $C_1—C_2$-Alkoxy substituiertes $C_1—C_6$-Alkyl, oder aber $C_3—C_4$-Alkenyl oder $C_3—C_4$-Alkinyl bedeutet, $R_8$ Wasserstoff oder $C_1—C_3$-Alkyl, $R_9$ $C_1—C_3$-Alkyl oder Phenyl, $R_{10}$ $C_1—C_4$-Alkyl oder Mono- oder Di($C_1—C_3$)-Alkylamin und $R_{11}$ gegebenenfalls durch $C_1—C_2$-Alkoxy substituiertes $C_1—C_3$-Alkyl darstellen, wobei im Falle $R_3 = —CH(CH_3)COOR_4$ der Substituent $R_7$ auch $C_1—C_6$-Alkyl bedeuten kann.

2. Mittel gemäss Anspruch 1, enthaltend mindestens eine Verbindung der Formel I als Wirkstoff, worin

$R_2$ Wasserstoff oder Methyl ist,

$R_3 = -CH(CH_3)COOR_4$ oder 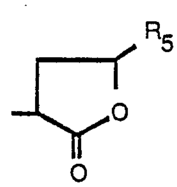

bedeutet,

$R_4$ gegebenenfalls durch $C_1—C_2$-Alkoxy substituiertes $C_1—C_3$-Alkyl oder aber $C_2—C_4$-Alkenyl oder $C_3—C_4$-Alkinyl darstellt,

$R_5$ Wasserstoff oder Methyl ist und

$R_6 = $ 2-Furyl, 2-Tetrahydrofuryl oder $CH_2Z$ darstellt, mit einer Bedeutung für Z von

a) $—X—R_7$, worbei X Sauerstoff oder Schwefel ist,

b) $—NH—N(R_8)(R_9)$,

c) $—OSO_2R_{10}$,

d) 1,2,4-Triazolyl (1) und dessen Salze und Metallkomplexe, wobei

$R_7$ im Falle von $R_3 = $ Lacton entweder Allyl oder Propargyl bedeutet, und

$R_7$ im Falle von $R_3 = —CH(CH_3)COOR_4$ wahlweise Allyl, Propargyl oder $C_1—C_3$-Alkyl bedeutet,

$R_8$ und $R_9$ unabhängig $C_1—C_2$-Alkyl bedeuten und

$R_{10}$ $C_1—C_2$-Alkyl oder Monomethylamin

darstellt.

3. Mittel gemäss Anspruch 1, enthaltend mindestens eine Verbindung der Formel I als Wirkstoff, worin

$R_2$ Wasserstoff oder Methyl ist,

$R_3$ = $-CH(CH_3)-COOR_4$ oder 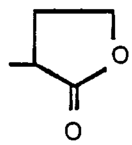

bedeutet, wobei

$R_4$ $C_1$—$C_3$-Alkyl darstellt,

$R_6$ = 2-Furyl, 2-Tetrahydrofuryl oder —$CH_2Z$ bedeutet, wobei Z im Falle von $R_3$ = —$CH(CH_3)COOR_4$ wahlweise

a) —$OR_7$ mit $R_7$ = $C_1$—$C_3$-Alkyl bedeutet, oder

b) —$OSO_2R_{10}$ bedeutet, wobei $R_{10} = C_1$—$C_2$-Alkyl oder Monomethylamin ist, oder

c) 1,2,4-Triazolyl (1) darstellt, und wobei Z im Falle $R_3$ = Lacton eine der beiden vorstehenden Bedeutungen b) oder c) hat.

4. Mittel gemäss Anspruch 1, enthaltend mindestens eine Verbindung aus der Gruppe
N-(2-Propin-1-yloxyacetyl)-N-(2-methylnaphthyl)-N-(2-oxo-tetrahydrofuran-3-yl)-amin,
N-(2-Methylnaphthyl)-N-(2-propin-1-yl-oxyacetyl)-alaninmethylester oder
N-(2-Methylnaphthyl)-N-(2-propin-1-yl-oxyacetyl)-alaninisopropylester als Wirkstoff.

5. Mittel gemäss Anspruch 1, enthaltend mindestens die Verbindung N-(2-Propen-1-yloxyacetyl)-N-(2-methylnaphthyl)-N-(2-oxo-tetrahydrofuran-3-yl)-amin als Wirkstoff.

6. Mittel gemäss Anspruch 1, enthaltend mindestens eine Verbindung aus der Gruppe
N-(2-Methylnaphthyl)-N-methoxyacetyl-alanin-2-methoxyäthylester,
N-(2-Methylnaphthyl)-N-methoxyacetyl-alanin-2-isopropylester,
N-(2,3-Dimethylnaphthyl)-N-methoxyacetyl-alaninmethylester als Wirkstoff.

7. Mittel gemäss Anspruch 1, enthaltend mindestens die Verbindung
N-(2-Methylnaphthyl)-N-(isopropoxyacetyl)-alaninmethylester als Wirkstoff.

8. Mittel gemäss Anspruch 1, enthaltend mindestens die Verbindung
N-(2,3-Dimethylnaphthyl)-N-äthoxyacetyl-alaninmethylester als Wirkstoff.

9. Mittel gemäss Anspruch 1, enthaltend mindestens die Verbindung
N-(2,3-Dimethylnaphthyl)-N-(β-methoxy-äthoxyacetyl)-N-(2-oxotetrahydrofuran-3-yl)-amin als Wirkstoff.

10. Mittel gemäss Anspruch 1, enthaltend mindestens eine Verbindung aus der Gruppe
N-(2-Methylnaphthyl)-N-methylsulfonyloxyacetyl-alaninmethylester,
N-(2-Methylnaphthyl)-N-methylsulfonyloxyacetyl-alanisopropylester,
N-Methylsulfonyloxyacetyl-N-(2-methylnaphthyl)-N-(2-oxo-tetrahydrofuran-3-yl)-amin,
N-(2,3-Dimethylnaphthyl)-N-(methylsulfonyloxyacetyl)-N-(2-oxo-tetrahydrofuran-3-yl)-amin als Wirkstoff.

11. Mittel gemäss Anspruch 1, enthaltend mindestens eine Verbindung aus der Gruppe
N-(2-Methylnaphthyl)-N-(N'-methylsulfamoyloxyacetyl)-alanin-methylester,
N-(2,3-Dimethylnaphthyl)-N-(N'-methylsulfamoyloxyacetyl)-alanin-methyester,
N-(N'-Methylsulfamoyloxyacetyl)-N-(2-methylnaphthyl)-N-(2-oxo-tetrahydrofuran-3-yl)-amin,
N-(2,3-Dimethylnaphthyl)-N-(N'-methylsulfamoyloxyacetyl)-N-(2-oxo-tetrahydrofuran-3-yl)-amin als Wirkstoff.

12. Mittel gemäss Anspruch 1, enthaltend mindestens eine Verbindung aus der Gruppe
N-(2-Methylnaphthyl)-N-[1,2,4-triazolyl (1)-acetyl]-alaninmethylester,
N-(2-Methylnaphthyl)-N-[1,2,4-triazolyl (1)-acetyl]-alanin-2-methoxyäthylester,
N-(2-Methylnaphthyl)-N-[1,2,4-triazolyl (1)-acetyl]-alaninisopropylester,
N-(2,3-Dimethylnaphthyl)-N-[1,2,4-triazolyl (1)-acetyl]-alaninmethylester,
N-(2-Methylnaphthyl)-N-(2-oxo-tetrahydrofuran-3-yl)-N-[1,2,4-triazolyl-(1)-acetyl]-amin,
N-(2-Methylnaphthyl)-N-(2-oxo-5-methyl-tetrahydrofuran-3-yl)-N-[1,2,4-triazolyl (1)-acetyl]-amin,
N-(2,3-Dimethylnaphthyl)-N-(2-oxo-tetrahydrofuran-3-yl)-N-[1,2,4-triazolyl (1)-acetyl]-amin als Wirkstoff.

13. Mittel gemäss Anspruch 1, enthaltend mindestens eine Verbindung aus der Gruppe
N-(2-Methylnaphthyl)-N-(2-oxo-tetrahydrofuran-3-yl)-N-[tetrahydrofuran-3-yl)-N-[tetrahydrofuranoyl (2)]-amin,
N-(2,3-Dimethylnaphthyl)-N-(2-oxo-tetrahydrofuran-3-yl)-N-[tetrahydrofuranoyl (2)]-amin,
N-(2-Methylnaphthyl)-N-[tetrahydrofuranoyl (2)]-alaninmethylester,
N-(2-Methylnaphthyl)-N-[tetrahydrofuranoyl (2)]-alanin-3-methoxyäthylester,
N-(2-Methylnaphthyl)-N-[tetrahydrofuranoyl (2)]-alaninisopropylester,
N-(2,3-Dimethylnaphthyl)-N-[tetrahydrofuranoyl (2)]-alaninmethylester als Wirkstoff.

14. Mittel gemäss Anspruch 1, enthaltend mindestens eine Verbindung aus der Gruppe
N-(2,3-Dimethylnaphthyl)-N-[furanoyl (2)]-alaninmethylester,
N-(2-Methylnaphthyl)-N-[furanoyl (2)]-alanin-2-methoxyäthylester als Wirkstoff.

15. Mittel gemäss Anspruch 1, enthaltend mindestens die Verbindung N-(2-Methylnaphthyl-N-(N'-2-phenylhydrazinoacetyl)-alaninmethylester als Wirkstoff.

16. Mittel gemäss Anspruch 1, enthaltend mindestens die Verbindung aus der Gruppe N-(2-Methylnaphthyl)-N-(N',N'-2-dimethylhydrazinoacetyl)-alaninmethylester, N-(2,3-Dimethylnaphthyl-N-(N',N'-dimethylhydrazinoacetyl)-alaninmethylester als Wirkstoff.

17. Verfahren zur Herstellung von N-acylierten alpha-Naphthylaminen der Formel I gemäss Anspruch 1, dadurch gekennzeichnet, dass man

A. eine Verbindung der Formel II mit einer Carbonsäure der Formel III oder ihrem Säurehalogenid oder Säureanhydrid acyliert

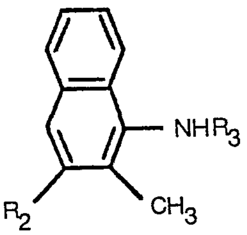

(II)

$HO-CO-R_6$ ,

(III)

oder dass man

B. eine Verbindung der Formel IV oder ihr Salz mit wahlweise einer Verbindung der Formel V, VI oder VII zur Reaktion bringt,

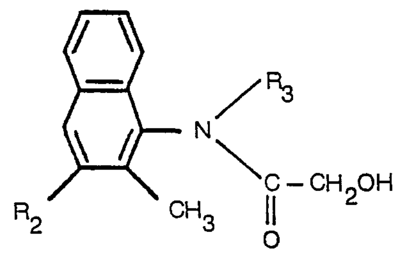

(IV)

+

$Hal-SO_2R_{10}$ (V)

$Hal-CO-R_{11}$ (VI)

$Hal-R_7$ (VII)

oder dass man

C. eine Verbindung der Formel X wahlweise mit einer Verbindung der Formel XI, XII, XIII oder XIIIa zur Reaktion bringt,

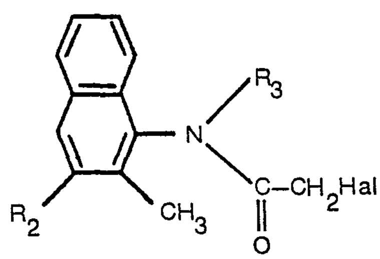

(X)

+

$MXR_7$ (XI)

$MO(CO)R_{11}$ (XII)

$NH_2N(R_8)(R_9)$ (XIII)

M–Azolyl (XIIIa)

wobei in den vorstehend genannten Formeln die Substituenten $R_2$, $R_3$, $R_6$ bis $R_{11}$ und X die für Formel I angegebenen Bedeutungen haben, während "Hal" für Halogen, "M" Wasserstoff oder ein Metallkation, bedeutet, und "Azolyl" ein 1,2-Pyrazolyl (1) oder 1,2,4-Triazolyl (1) darstellt.

18. Verfahren gemäss Anspruch 17, dadurch gekennzeichnet, dass man eine der Verbindungen der Formel I gemäss einem der Ansprüche 2 bis 16 herstellt.

19. Verwendung einer Verbindung der Formel I des Anspruchs 1 zur Bekämpfung oder zur Verhütung von Pilzbefall an Pflanzen.

20. Verwendung einer Verbindung des Formel I des Anspruchs 1 gemäss einem der Ansprüche 2 bis 16, gemaß Anspruch 19.

**Claims for the Contracting States: BE CH DE FR GB IT LI NL SE**

1. An acylated naphthylamine of the formula I

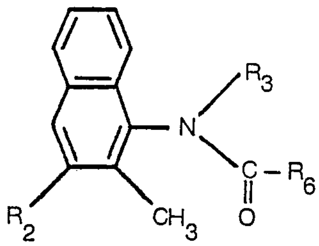

(I)

wherein $R_2$ is hydrogen or methyl; $R_3$ is $-CH(CH_3)COOR_4$ or

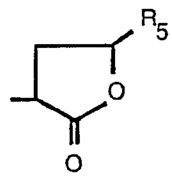

in which $R_4$ is $C_1-C_4$-alkyl, $C_2-C_4$-alkenyl, $C_3-C_4$-alkynyl or $C_3-C_7$-cycloalkyl, each of which is unsubstituted or is substituted by halogen or by $C_1-C_2$-alkoxy, and $R_5$ is hydrogen or methyl; and $R_6$ is 2-furyl or 2-tetrahydrofuryl each of which is unsubstituted or is substituted by halogen, or $R_6$ is $\beta$-($C_1-C_4$)-alkoxyethyl or the group $CH_2Z$, where Z is one of the groups a) $-X-R_7$, b) $-NH-N(R_8)(R_9)$, c) $-OSO_2R_{10}$, d) $-O(CO)R_{11}$, e) 1,2-pyrazolyl (1) or f) 1,2,4-triazolyl (1), including the salts and metal complexes thereof, and X is oxygen or sulfur, $R_7$ is a $C_1-C_6$-alkyl group substituted by $C_1-C_2$-alkoxy, or it is $C_3-C_4$-alkenyl or $C_3-C_4$-alkynyl, $R_8$ is hydrogen or $C_1-C_3$-alkyl, $R_9$ is $C_1-C_3$-alkyl or phenyl, $R_{10}$ is $C_1-C_4$-alkyl or mono- or di-($C_1-C_3$)-alkylamine, and $R_{11}$ is $C_1-C_3$-alkyl which is unsubstituted or is substituted by $C_1-C_2$-alkoxy; and in the case where $R_3$ is $-CH(CH_3)COOR_4$, the substituent $R_7$ can also be $C_1-C_6$-alkyl.

2. An acylated naphthylamine of the formula I according to Claim 1, wherein

$R_2$ is hydrogen or methyl,

$R_3$ is $-CH(CH_3)COOR_4$ or 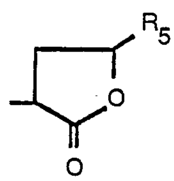 ,

$R_4$ is $C_1-C_3$-alkyl which is unsubstituted or substituted by $C_1-C_2$-alkoxy, or $R_4$ is $C_2-C_4$-alkenyl or $C_3-C_4$-alkynyl,

$R_5$ is hydrogen or methyl, and

$R_6$ is 2 furyl, 2 tetrahydrofuryl or $CH_2Z$, where Z is one of the groups

a) $-X-R_7$, wherein X is oxygen or sulfur,

b) $-NH-N(R_8)(R_9)$,

c) $-OSO_2R_{10}$,

d) 1,2,4-triazolyl (1) and salts and metal complexes thereof, and

$R_7$ in the case where $R_3$ is lactone is either allyl or propargyl, and

$R_7$ in the case where $R_3$ is $-CH(CH_3)COOR_4$ is allyl, propargyl or $C_1-C_3$-alkyl,

$R_8$ and $R_9$ independently of one another are each $C_1-C_2$-alkyl, and

$R_{10}$ is $C_1-C_2$-alkyl or monomethylamine.

3. An acylated naphthylamine of the formula I according to Claim 1, wherein

$R_2$ is hydrogen or methyl,

$R_3$ is $-CH(CH_3)-COOR_4$ or 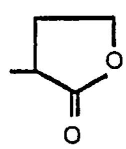 ,

wherein

$R_4$ is $C_1-C_3$-alkyl,

$R_6$ is 2-furyl, 2-tetrahydrofuryl or $—CH_2Z$, wherein
Z in the case where $R_3$ is $—CH(CH_3)COOR_4$ is
a) $—OR_7$, where $R_7$ is $C_1—C_3$-alkyl, or
b) $—OSO_2R_{10}$, where $R_{10}$ is $C_1—C_2$-alkyl or monomethylamine or
c) 1,2,4-triazolyl (1), and
Z in the case where $R_3$ is lactone has one of the two aforementioned meanings b) and c).

4. Any compound selected from the group comprising:
N-(2-propyn-1-yloxyacetyl)-N-(2-methylnaphthyl)-N-(2-oxo-tetrahydrofuran-3-yl) amine,
N-(2-methylnaphthyl)-N-(2-propyn-1-yl-oxyacetyl)-alaninemethyl ester, and
N-(2-methylnaphthyl)-N-(2-propyn-1-yl-oxyacetyl)-alanineisopropyl ester.

5. N-(2-Propen-1-yloxyacetal)-N-(2-methylnaphthyl)-N-(2-oxo-tetrahydrofuran-3-yl) amine.

6. Any compound selected from the group comprising:
N-(2-methylnaphthyl)-N-methoxyacetyl-alanine-2-methoxyethyl ester,
N-(2-methylnaphthyl)-N-methoxyacetyl-alanine-2-isopropyl ester, and
N-(2,3-dimethylnaphthyl)-N-methoxyacetyl-alaninemethyl ester.

7. N-(2-Methylnaphthyl)-N-(isopropoxyacetyl)-alaninemethyl ester.

8. N-(2,3-Dimethylnaphthyl)-N-ethoxyacetyl-alaninemethyl ester.

9. N-(2,3-Dimethylnaphthyl)-N-($\beta$-methoxy-ethoxyacetyl)-N-(2-oxotetrahydrofuran-3-yl)-amine.

10. Any compound selected from the group comprising:
N-(2-methylnaphthyl)-N-methylsulfonyloxyacetyl-alaninemethyl ester,
N-(2-methylnaphthyl)-N-methylsulfonyloxyacetyl-alanineisopropyl ester,
N-methylsulfonyloxyacetyl-N-(2-methylnaphthyl)-N-(2-oxo-tetrahydrofuran-3-yl) amine, and
N-(2,3-dimethylnaphthyl)-N-(methylsulfonyloxyacetyl)-N-(2-oxo-tetrahydrofuran-3-yl) amine.

11. Any compound selected from the group comprising:
N-(2-methylnaphthyl)-N-(N'-methylsulfamoyloxyacetyl)-alaninemethyl ester,
N-2,3-dimethylnaphthyl)-N-(N'-methylsulfamoyloxyacetyl)-alanine-methyl ester,
N-(N'-methylsulfamoyloxyacetyl)-N-(2-methylnaphthyl)-N-(2-oxo-tetrahydrofuran-3-yl) amine, and
N-(2,3-dimethylnaphthyl)-N-(N'-methylsulfamoyloxyacetyl)-N-(2-oxo-tetrahydrofuran-3-yl) amine.

12. Any compound selected from the group comprising:
N-(2-methylnaphthyl)-N-[1,2,4-triazolyl(1)-acetyl]-alanine-methyl ester,
N-(2-methylnaphthyl)-N-[1,2,4-triazolyl(1)-acetyl]-alanine-2-methoxyethyl ester,
N-(2-methylnaphthyl)-N-[1,2,4-triazolyl(1)-acetyl]-alanine, isopropyl ester,
N-(2,3-dimethylnaphthyl)-N-[1,2,4-triazolyl(1)-acetyl]-alanine-methyl ester,
N-(2-methylnaphthyl)-N-(2-oxo-tetrahydrofuran-3-yl)-N-[1,2,4-triazolyl(1)-acetyl] amine,
N-(2-methylnaphthyl)-N-(2-oxo-5-methyl-tetrahydrofuran-3-yl)-N-[1,2,4-triazolyl(1)-acetyl] amine, and
N-(2,3-dimethylnaphthyl)-N-(2-oxo-tetrahydrofuran-3-yl)-N-[1,2,4-triazolyl(1)-acetyl] amine.

13. Any compound selected from the group comprising:
N-(2-methylnaphthyl)-N-(2-oxo-tetrahydrofuran-3-yl)-N-[tetrahydrofuran-3-yl)-N-[tetrahydrofuranoyl(2)] amine,
N-(2,3-dimethylnaphthyl)-N-(2-oxo-tetrahydrofuran-3-yl)-N-[tetrahydrofuranoyl(2)] amine,
N-(2-methylnaphthyl)-N-[tetrahydrofuranoyl(2)]-alanine-methyl ester,
N-(2-methylnaphthyl)-N-[tetrahydrofuranoyl(2)]-alanine-3-methoxyethyl ester,
N-(2-methylnaphthyl)-N-[tetrahydrofuranoyl(2)]-alanine-isopropyl ester, and
N-(2,3-dimethylnaphthyl)-N-[tetrahydrofuranoyl(2)]-alanine-methyl ester.

14. Any compound selected from the group comprising:
N-(2,3-dimethylnaphthyl)-N-[furanoyl(2)]-alanine-methyl ester, and
N-(2-methylnaphthyl)-N-[furanoyl(2)]-alanine-2-methoxyethyl ester.

15. N-(2-Methylnaphthyl-N-(N'-2-phenylhydrazinoacetyl)-alanine-methyl ester.

16. Any compound selected from the group comprising:
N-(2-methylnaphthyl)-N-(N',N'-2-dimethylhydrazinoacetyl)-alanine-methyl ester, and
N-(2,3-dimethylnaphthyl-N-(N',N'-2-dimethylhydrazinoacetyl)-alanine-methyl ester.

17. A process for producing an acylated naphthylamine of the formula I according to Claim 1, which process comprises

A. acylating a compound of the formula II with a carboxylic acid of the formula III or with an acid halide or acid anhydride thereof

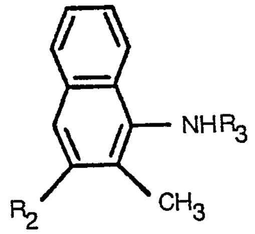

$HO—CO—R_6$ ,

(II) (III)

B. reacting a compound of the formula IV or a salt thereof with a compound of the formula V, VI or VII:

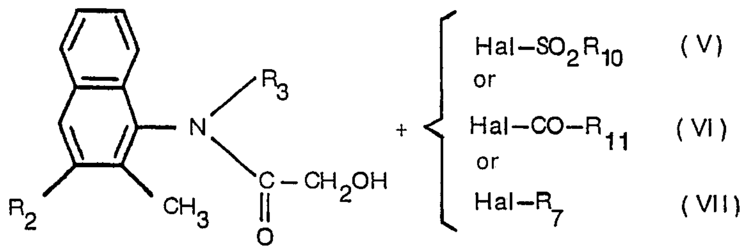


(IV)

or

C. reacting a compound of the formula X with a compound of the formula XI, XII, XIII or XIIIa:

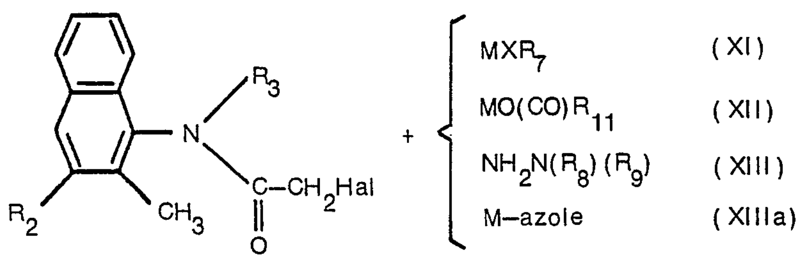


(X)

where in the formulae given above the substituents $R_2$, $R_3$, $R_6$ to $R_{11}$ and X have the meanings defined under the formula I, while "Hal" is halogen, preferably chlorine or bromine, "M" is hydrogen or a metal cation, preferably an alkali metal cation or alkaline-earth metal cation, and "azole" is a 1,2-pyrazolyl(1) or 1,2,4-triazolyl(1).

18. A plant fungicidal composition containing as active substance at least one compound of the formula I according to Claim 1, together with a suitable carrier and/or a surface-active agent.

19. A composition according to Claim 18 containing as active substance a compound of the formula I according to any one of Claims 2 to 16.

20. Use of a compound of the formula I of Claim 1 for combating or preventing fungus infection on plants.

21. Use according to Claim 20 of a compound of the formula I of Claim 1 as claimed in any one of Claims 2 to 16.

**Claims for the Contracting State: AT**

1. A plant fungicidal composition containing as active substance at least one N-acylated alpha-naphthylamine of the general formula I

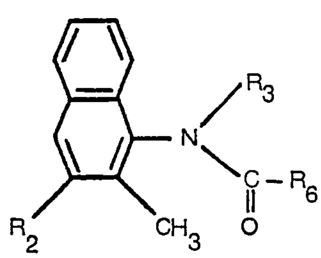

(I)

wherein $R_2$ is hydrogen or methyl; $R_3$ is $-CH(CH_3)COOR_4$ or

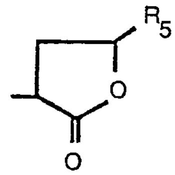

in which $R_4$ is $C_1$—$C_4$-alkyl, $C_2$—$C_4$-alkenyl, $C_3$—$C_4$-alkynyl or $C_3$—$C_7$-cycloalkyl, each of which is unsubstituted or is substituted by halogen or by $C_1$—$C_2$-alkoxy, and $R_5$ is hydrogen or methyl; and $R_6$ is 2-furyl or 2-tetrahydrofuryl each of which is unsubstituted or is substituted by halogen, or $R_6$ is $\beta$-($C_1$—$C_4$)-alkoxyethyl or the group $CH_2Z$, where Z is one of the groups a) —X—$R_7$, b) —NH—N($R_8$)($R_9$), c) —$OSO_2R_{10}$, d) —O(CO)$R_{11}$, e) 1,2-pyrazolyl (1) or f) 1,2,4-triazolyl (1), including the salts and metal complexes thereof, and X is oxygen or sulfur, $R_7$ is a $C_1$—$C_6$-alkyl group substituted by $C_1$—$C_2$-alkoxy, or it is $C_3$—$C_4$-alkenyl or $C_3$—$C_4$-alkynyl, $R_8$ is hydrogen or $C_1$—$C_3$-alkyl, $R_9$ is $C_1$—$C_3$-alkyl or phenyl, $R_{10}$ is $C_1$—$C_4$-alkyl or mono- or di-($C_1$—$C_3$)-alkylamine, and $R_{11}$ is $C_1$—$C_3$-alkyl which is unsubstituted or is substituted by $C_1$—$C_2$-alkoxy; and in the case where $R_3$ is —$CH(CH_3)COOR_4$, the substituent $R_7$ can also be $C_1$—$C_6$-alkyl.

2. A composition according to Claim 1 containing as active substance at least one compound of the formula I wherein

$R_2$ is hydrogen or methyl,

$R_3$ is $-CH(CH_3)-COOR_4$ or 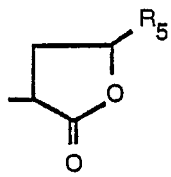 ,

$R_4$ is $C_1$—$C_3$-alkyl which is unsubstituted or substituted by $C_1$—$C_2$-alkoxy, or $R_4$ is $C_2$—$C_4$-alkenyl or $C_3$—$C_4$-alkynyl,

$R_5$ is hydrogen or methyl, and

$R_6$ is 2 furyl, 2 tetrahydrofuryl or $CH_2Z$, where Z is one of the groups

a) —X—$R_7$, wherein X is oxygen or sulfur,

b) —NH—N($R_8$)($R_9$),

c) —$OSO_2R_{10}$,

d) 1,2,4-triazolyl (1) and salts and metal complexes thereof, and

$R_7$ in the case where $R_3$ is lactone is either allyl or propargyl, and

$R_7$ in the case where $R_3$ is —$CH(CH_3)COOR_4$ is allyl, propargyl or $C_1$—$C_3$-alkyl,

$R_8$ and $R_9$ independently of one another are each $C_1$—$C_2$-alkyl, and

$R_{10}$ is $C_1$—$C_2$-alkyl or monomethylamine.

3. A composition according to Claim 1 containing as active substance at least one compound of the formula I wherein

$R_2$ is hydrogen or methyl,

$R_3$ is $-CH(CH_3)-COOR_4$ or 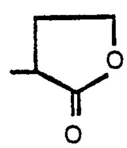 ,

wherein

$R_4$ is $C_1$—$C_3$-alkyl,

$R_6$ is 2-furyl, 2-tetrahydrofuryl or —$CH_2Z$, wherein

Z in the case where $R_3$ is —$CH(CH_3)COOR_4$ is

a) —$OR_7$, where $R_7$ is $C_1$—$C_3$-alkyl, or

b) —$OSO_2R_{10}$, where $R_{10}$ is $C_1$—$C_2$-alkyl or monomethylamine or

c) 1,2,4-triazolyl (1), and

Z in the case where $R_3$ is lactone has one of the two aforementioned meanings b) and c).

4. A composition according to Claim 1 containing as active substance at least one compound selected from the group comprising:

N-(2-propyn-1-yloxyacetyl)-N-(2-methylnaphthyl)-N-(2-oxo-tetrahydrofuran-3-yl) amine,

N-(2-methylnaphthyl)-N-(2-propyn-1-yl-oxyacetyl)-alaninemethyl ester, and

N-(2-methylnaphthyl)-N-(2-propyn-1-yl-oxyacetyl)-alanineisopropyl ester.

5. A composition according to Claim 1 containing as active substance at least the compound N-(2-propen-1-yloxyacetyl)-N-(2-methylnaphthyl)-N-(2-oxo-tetrahydrofuran-3-yl) amine.

6. A composition according to Claim 1 containing as active substance at least one compound selected from the group comprising:

N-(2-methylnaphthyl)-N-methoxyacetyl-alanine-2-methoxyethyl ester,

N-(2-methylnaphthyl)-N-methoxyacetyl-alanine-2-isopropyl ester, and
N-(2,3-dimethylnaphthyl)-N-methoxyacetyl-alaninemethyl ester.

7. A composition according to Claim 1 containing as active substance at least the compound N-(2-methylnaphthyl)-N-(isopropoxyacetyl)-alaninemethyl ester.

8. A composition according to Claim 1 containing as active substance at least the compound N-(2,3-dimethylnaphthyl)-N-ethoxyacetyl-alaninemethyl ester.

9. A composition according to Claim 1 containing as active substance at least the compound N-(2,3-dimethylnaphthyl)-N-($\beta$-methoxy-ethoxyacetyl)-N-(2-oxo-tetrahydrofuran-3-yl)-amine.

10. A composition according to Claim 1 containing as active substance at least one compound selected from the group comprising:
N-(2-methylnaphthyl)-N-methylsulfonyloxyacetyl-alaninemethyl ester,
N-(2-methylnaphthyl)-N-methylsulfonyloxyacetyl-alanineisopropyl ester,
N-methylsulfonyloxyacetyl-N-(2-methylnaphthyl)-N-(2-oxo-tetrahydrofuran-3-yl) amine, and
N-(2,3-dimethylnaphthyl)-N-(methylsulfonyloxyacetyl)-N-(2-oxo-tetrahydrofuran-3-yl) amine.

11. A composition according to Claim 1 containing as active substance at least one compound selected from the group comprising
N-(2-methylnaphthyl)-N-(N′-methylsulfamoyloxyacetyl)-alaninemethyl ester,
N-(2,3-dimethylnaphthyl)-N-(N′-methylsulfamoyloxyacetyl)-alanine-methyl ester,
N-(N′-methylsulfamoyloxyacetyl)-N-(2-methylnaphthyl)-N-(2-oxo-tetrahydrofuran-3-yl) amine, and
N-(2,3-dimethylnaphthyl)-N-(N′-methylsulfamoyloxyacetyl)-N-(2-oxo-tetrahydrofuran-3-yl) amine.

12. A composition according to Claim 1 containing as active substance at least one compound selected from the group comprising:
N-(2-methylnaphthyl)-N-[1,2,4-triazolyl(1)-acetyl]-alanine-methyl ester,
N-(2-methylnaphthyl)-N-[1,2,4-triazolyl(1)-acetyl]-alanine-2-methoxyethyl ester,
N-(2-methylnaphthyl)-N-[1,2,4-triazolyl(1)-acetyl]-alanine, isopropyl ester,
N-(2,3-dimethylnaphthyl)-N-[1,2,4-triazolyl(1)-acetyl]-alanine-methyl ester,
N-(2-methylnaphthyl)-N-(2-oxo-tetrahydrofuran-3-yl)-N-[1,2,4-triazolyl(1)-acetyl] amine,
N-(2-methylnaphthyl)-N-(2-oxo-5-methyl-tetrahydrofuran-3-yl)-N-[1,2,4-triazolyl(1)-acetyl] amine, and
N-(2,3-dimethylnaphthyl)-N-(2-oxo-tetrahydrofuran-3-yl)-N-[1,2,4-triazolyl(1)-acetyl] amine.

13. A composition according to Claim 1 containing as active substance at least one compound selected from the group comprising:
N-(2-methylnaphthyl)-N-(2-oxo-tetrahydrofuran-3-yl)-N-[tetrahydrofuran-3-yl)-N-[tetrahydrofuranoyl(2)] amine,
N-(2,3-dimethylnaphthyl)-N-(2-oxo-tetrahydrofuran-3-yl)-N-[tetrahydrofuranoyl(2)] amine,
N-(2-methylnaphthyl)-N-[tetrahydrofuranoyl(2)]-alanine-methyl ester,
N-(2-methylnaphthyl)-N-[tetrahydrofuranoyl(2)]-alanine-3-methoxyethyl ester,
N-(2-methylnaphthyl)-N-[tetrahydrofuranoyl(2)]-alanine-isopropyl ester, and
N-(2,3-dimethylnaphthyl)-N-[tetrahydrofuranoyl(2)]-alanine-methyl ester.

14. A composition according to Claim 1 containing as active substance at least one compound selected from the group comprising:
N-(2,3-dimethylnaphthyl)-N-[furanoyl(2)]-alanine-methyl ester, and
N-(2-methylnaphthyl)-N-[furanoyl(2)]-alanine-2-methoxyethyl ester.

15. A composition according to Claim 1 containing as active substance at least the compound N-(2-methylnaphthyl-N-(N′-2-phenylhydrazinoacetyl)-alanine-methyl ester.

16. A composition according to Claim 1 containing as active substance at least one compound selected from the group comprising:
N-(2-methylnaphthyl)-N-(N′,N′-2-dimethylhydrazinoacetyl)-alanine-methyl ester, and
N-(2,3-dimethylnaphthyl-N-(N′,N′-2-dimethylhydrazinoacetyl)-alanine-methyl ester.

17. A process for producing an N-acylated alpha-naphthylamine of the formula I according to Claim 1, which process comprises

A. acylating a compound of the formula II with a carboxylic acid of the formula III or with an acid halide or acid anhydride thereof

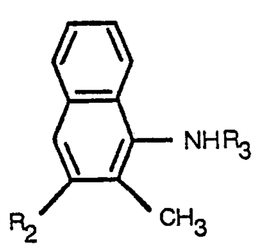

$HO-CO-R_6$ ,

(II) (III)

B. reacting a compound of the formula IV or a salt thereof with a compound of the formula V, VI or VII:

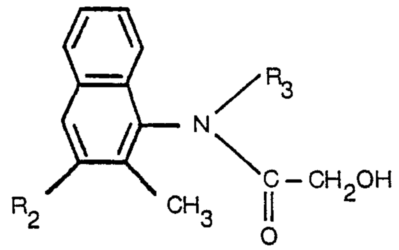

(IV)

\+

| | |
|---|---|
| $Hal-SO_2R_{10}$ | (V) |
| or | |
| $Hal-CO-R_{11}$ | (VI) |
| or | |
| $Hal-R_7$ | (VII) |

or

C. reacting a compound of the formula X with a compound of the formula XI, XII, XIII or XIIIa:

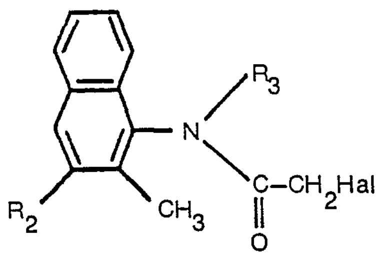

(X)

\+

| | |
|---|---|
| $MXR_7$ | (XI) |
| $MO(CO)R_{11}$ | (XII) |
| $NH_2N(R_8)(R_9)$ | (XIII) |
| M—azole | (XIIIa) |

where in the formulae given above the substituents $R_2$, $R_3$, $R_6$ to $R_{11}$ and X have the meanings defined under the formula I, while "Hal" is halogen, "M" is hydrogen or a metal cation, and "azole" is a 1,2-pyrazolyl(1) or 1,2,4-triazolyl(1).

18. A process according to Claim 17, whereby any one of the compounds of the formula I according to any one of Claims 2 to 16 is produced.

19. Use of a compound of the formula I of Claim 1 for combating or preventing fungus infection on plants.

20. Use according to Claim 19 of a compound of the formula I of Claim 1 as claimed in any one of Claims 2 to 16.

**Revendications pour les Etats contractants: BE CH DE FR GB IT LI NL SE**

1. Dérivés de naphtylamine acylés de formule I

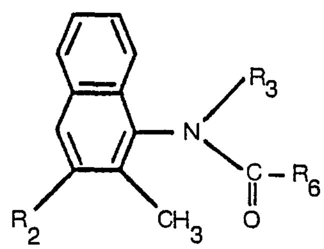

(I)

où $R_2$ représente un hydrogène ou un méthyle, $R_3$ représente —$CH(CH_3)COOR_4$ ou

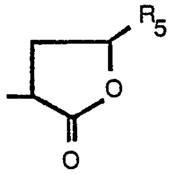

où $R_4$ représente un alcoyle en $C_1$ à $C_4$, alcényle en $C_2$ à $C_4$, alcynyle en $C_3$ à $C_4$ ou cycloalcoyle en $C_3$ à $C_7$ à substitution éventuelle, halogène ou alcoxy en $C_1$ à $C_2$ et $R_5$ représente un hydrogène ou un méthyle, et $R_6$ représente un 2-furyl- ou un 2-tétrahydrofuryle à substitution éventuelle, halogène, un $\beta$-alcoxy en $C_1$ à $C_4$-éthyle ou le groupe $CH_2Z$, où Z représente l'un des groupes a) —X—$R_7$, b)

—NH—$N(R_8)(R_9)$, c) —$OSO_2R_{10}$, d) —$(CO)R_{11}$, e) 1,2-pyrazolyle(1) ou f) 1,2,4-triazolyle(1), y compris leurs sels et complexes métalliques, et X est un oxygène ou un soufre, $R_7$ est un alcoyle en $C_1$ à $C_6$ substitué par un alcoxy en $C_1$ à $C_2$, ou encore un alcényle en $C_3$ à $C_4$ ou un alcynyle en $C_3$ à $C_4$, $R_8$ représente un hydrogène ou un alcoyle en $C_1$ à $C_3$, $R_9$ représente un alcoyle en $C_1$ à $C_3$ ou un phényle, $R_{10}$ représente un alcoyle en $C_1$ à $C_4$ ou une mono- ou di-alcoyle en $C_1$ à $C_3$-amine et $R_{11}$ représente un alcoyle en $C_1$ à $C_3$ à substitution éventuelle, alcoxy en $C_1$ à $C_2$, où si $R_3$ = —$CH(CH_3)COOR_4$ le substituant $R_7$ peut également représenter un alcoyle en $C_1$ à $C_6$.

2. Naphtylamines acylées de formule I selon la revendication 1, où $R_2$ représente un hydrogène ou un méthyle, $R_3$ = —$CH(CH_3)COOR_4$ ou

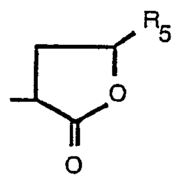

$R_4$ représente un alcoyle en $C_1$ à $C_3$ éventuellement substitué par un alcoxy en $C_1$ à $C_2$ ou encore un alcényle en $C_2$ à $C_4$ ou un alcynyle en $C_3$ à $C_4$,

$R_5$ est un hydrogène ou un méthyle et

$R_6$ représente un 2-furyle, un 2-tétrahydrofuryle ou $CH_2Z$, la signification de Z étant

a) —X—$R_7$, où X est un oxygène ou un soufre,

b) —NH—$N(R_8)(R_9)$,

c) —$OSO_2R_{10}$,

d) 1,2,4-triazolyle(1) et ses sels et complexes métalliques où $R_7$ si $R_3$ = lactone représente un allyle ou un propargyle, et

$R_7$ si $R_3$ = —$CH(CH_3)COOR_4$ représente au choix un allyle, un propargyle ou un alcoyle en $C_1$ à $C_3$,

$R_8$ et $R_9$ représentent de façon indépendante un alcoyle en $C_1$ à $C_2$ et

$R_{10}$ représente un alcoyle en $C_1$ à $C_2$ ou une monométhylamine.

3. Naphtylamines acylées de formule I selon la revendication 1, où $R_2$ est un hydrogène ou un méthyle,

$R_3$ = $-CH(CH_3)-COOR_4$ ou 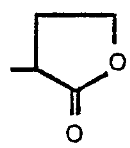

où

$R_4$ représente un alcoyle en $C_1$ à $C_3$,

$R_6$ = 2-furyle, 2-tétrahydrofuryle ou —$CH_2Z$, où

Z si $R_3$ = —$CH(CH_3)COOR_4$ représente au choix

a) —$OR_7$ avec $R_7$ = alcoyle en $C_1$ à $C_3$, ou

b) —$OSO_2R_{10}$, où $R_{10}$ = alcoyle en $C_1$ à $C_2$ ou représente une monométhylamine, ou

c) 1,2,4-triazolyle(1), et où Z si $R_3$ = lactone a l'une des deux significations b) ou c) précédentes.

4. Composé du groupe:

N-(2-propyn-1-yloxyacétyl)-N-(2-méthylnaphtyl)-N-(2-oxo-tétrahydrofuran-3-yl)-amine,
N-(2-méthylnaphtyl)-N-(2-propyn-1-yl-oxyacétyl)-alanine-méthylester ou
N-(2-méthylnaphtyl)-N-(2-propyn-1-yl-oxyacétyl)-alanine-propylester.

5. N-(2-propén-1-yloxyacétyl)-N-(2-méthylnaphtyl)-N-(2-oxo-tétrahydrofuran-3-yl)-amine.

6. Composé du groupe:

N-(2-méthylnaphtyl)-N-méthoxyacétyl-alanine-2-méthoxyéthylester,
N-(2-méthylnaphtyl)-N-méthoxyacétyl-alanine-2-isopropylester,
N-(2,3-diméthylnaphtyl)-N-méthoxyacétyl-alanineméthylester.

7. N-(2-méthylnaphtyl)-N-(isopropoxyacétyl)-alanine-méthylester.

8. N-(2,3-diméthylnaphtyl)-N-éthoxyacétyl-alanine-méthylester.

9. N-(2,3-diméthylnaphtyl)-N-($\beta$-méthoxy-éthoxyacétyl)-N-(2-oxotétrahydrofuran-3-yl)-amine.

10. Un des composés du groupe

N-(2-méthylnaphtyl)-N-méthylsulfonyloxyacétyl-alanine-méthylester
N-(2-méthylnaphtyl)-N-méthylsulfonyloxyacétyl-alanine-isopropylester,
N-méthylsulfonyloxyacétyl-N-(2-méthylnaphtyl)-N-(2-oxo-tétrahydrofuran-3-yl)-amine,
N-(2,3-diméthylnaphtyl)-N-(méthylsulfonyloxyacétyl)-N-(2-oxo-tétrahydrofuran-3-yl)-amine.

11. Composé du groupe

N-(2-méthylnaphtyl)-N-(N'-méthylsulfamoyloxyacétyl)-alanine-méthylester,
N-(2,3-diméthylnaphtyl)-N-(N'-méthylsulfamoyloxyacétyl)-alanine-méthylester,
N-(N'-méthylsulfamoyloxyacétyl)-N-(2-méthylnaphtyl)-N-(2-oxo-tétrahydrofuranne-3-yl)-amine,
N-(2,3-diméthylnaphtyl)-N-(N'-méthylsulfamoyloxyacétyl)-N-(2-oxo-tétrahydrofuran-3-yl)-amine.

12. Un des composés du groupe

N-(2-méthylnaphtyl)-N-[1,2,4-triazolyl(1)-acétyl]-alanineméthylester,
N-(2-méthylnaphtyl)-N-[1,2,4-triazolyl(1)-acétyl]-alanine-2-méthoxy-éthylester,
N-(2-méthylnaphtyl)-N-[1,2,4-triazolyl(1)-acétyl]-alanineisopropylester,
N-(2,3-diméthylnaphtyl)-N-[1,2,4-triazolyl(1)-acétyl]-alanine-méthylester,
N-(2-méthylnaphtyl)-N-(2-oxo-tétrahydrofuran-3-yl)-N-[1,2,4-triazolyl(1)-acétyl]-amine,
N-(2-méthylnaphtyl)-N-(2-oxo-5-méthyl-tétrahydrofuran-3-yl)-N-[1,2,4-triazolyl(1)-acétyl]-amine,
N-(2,3-diméthylnaphtyl)-N-(2-oxo-tétrahydrofuran-3-yl)-N-[1,2,4-triazolyl(1)-acétyl]-amine.

13. Un des composés du groupe:
N-(2-méthylnaphtyl)-N-(2-oxo-tétrahydrofuran-3-yl)-N-[tétrahydrofuran-3-yl) N-[tétrahydrofuranoyl(2)]-amine,
N-(2,3-diméthylnaphtyl)-N-(2-oxo-tétrahydrofuran-3-yl)-N-[tétrahydrofuranoyl(2)]-amine,
N-(2-méthylnaphtyl)-N-[tétrahydrofuranoyl(2)]-alanine-méthylester,
N-(2-méthylnaphtyl)-N-[tétrahydrofuranoyl(2)]-alanine-3-méthoxyéthylester,
N-(2-méthylnaphtyl)-N-[tétrahydrofuranoyl(2)]-alanine-isopropylester,
N-(2,3-diméthylnaphtyl)-N-[tétrahydrofuranoyl(2)]-alanine-méthylester.

14. Composé du groupe
N-(2,3-diméthylnaphtyl)-N-[furanoyl(2)]-alanine-méthylester,
N-(2-méthylnaphtyl)-N-[furanoyl(2)]-alanine-2-méthoxyéthylester.

15. N-(2-méthylnaphtyl-N-(N'-2-phénylhydrazinoacétyl)-alanineméthylester.

16. Composé du groupe
N-(2-méthylnaphtyl)-N-(N',N'-2-diméthylhydrazinoacétyl)-alanineméthylester,
N-(2,3-diméthylnaphtyl-N-(N',N'-2-diméthylhydrazinoacétyl)-alanine-méthylester.

17. Procédé de préparation de naphtylamines acylées de formule I selon la revendication 1, caractérisé en ce que
A. on acyle un composé de formule II avec un acide carboxylique de formule III ou son halogénure d'acide ou anhydride d'acide

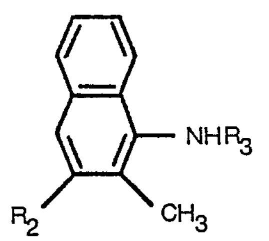

$HO-CO-R_6$ ,

(II) (III)

ou
B. on fait réagir un composé de formule IV ou son sel avec un choix un composé de formule V, VI ou VII,

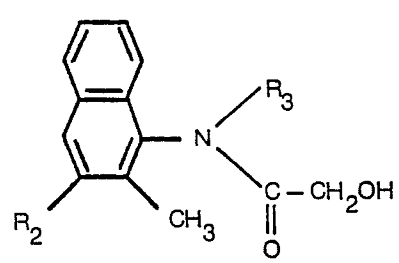

+

$Hal-SO_2R_{10}$ (V)
ou
$Hal-CO-R_{11}$ (VI)
ou
$Hal-R_7$ (VII)

(IV)

ou
C. on fait réagir un composé de formule X au choix avec un composé de formule XI, XII, XIII ou XIIIa,

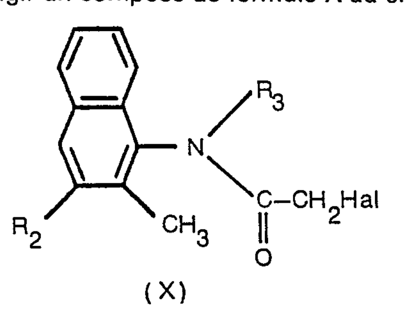

+

$MXR_7$ (XI)
$MO(CO)R_{11}$ (XII)
$NH_2N(R_8)(R_9)$ (XIII)
M-Azol (XIIIa)

(X)

où dans les formules mentionnées ci-dessus les substituants $R_2$, $R_3$, $R_6$ à $R_{11}$ et X ont les significations données pour la formule I, tandis que "Hal" représente un halogène, de préférence le chlore ou le brome, "M" représente un hydrogène ou un cation métallique, de préférence un cation alcalin ou alcalino-terreux, et "azolyle" représente un 1,2-pyrazolyle(1) ou un 1,2,4-triazolyle(1).

18. Agent fongicide pour plantes contenant comme matière active au moins un composé de formule I selon la revendication 1, avec un support et/ou additif tensioactif approprié.

19. Agent selon la revendication 18 contenant comme matière active un composé de formule I selon l'une des revendications 2 à 16.

20. Application d'un composé de formule I de la revendication 1 pour combattre ou prévenir l'attaque des champignons sur les plantes.

21. Application d'un composé de formule I de la revendication 1 selon l'une des revendications 2 à 16, selon la revendication 20.

**Revendications pour l'Etat contractant: AT**

1. Agent fongicide pour plantes contenant au moins une alpha-naphtylamine N-acylée de formule générale I comme matière active

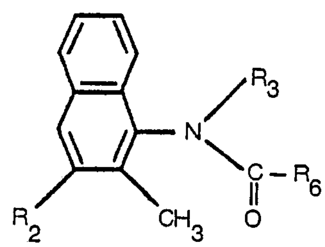

(I)

où $R_2$ représente un hydrogène ou un méthyle, $R_3$ représente —$CH(CH_3)COOR_4$ ou

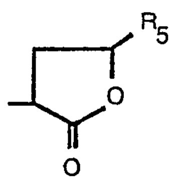

où $R_4$ représente un alcoyle en $C_1$ à $C_4$, alcényle en $C_2$ à $C_4$, alcynyle en $C_3$ à $C_4$ ou cycloalcoyle en $C_3$ à $C_7$ à substitution éventuelle, halogène ou alcoxy en $C_1$ à $C_2$ et $R_5$ représente un hydrogène ou un méthyle, et $R_6$ représente un 2-furyle ou 2-tétrahydrofuryle à substitution éventuelle, halogène, un $\beta$-alcoxy en $C_1$ à $C_4$-éthyle ou le groupe $CH_2Z$, où Z représente l'un des groupes a) —X—$R_7$, b) —NH—N($R_8$)($R_9$), c) —$OSO_2R_{10}$, d) —(CO)$R_{11}$, e) 1,2-pyrazolyle(1) ou f) 1,2,4-triazolyle(1), y compris leurs sels et complexes métalliques, et X est un oxygène ou un soufre, $R_7$ est un alcoyle en $C_1$ à $C_6$ substitution, alcoxy en $C_1$ à $C_2$, ou encore un alcényle en $C_3$ à $C_4$ ou un alcynyle en $C_3$ à $C_4$, $R_8$ représente un hydrogène ou un alcoyle en $C_1$ à $C_3$, $R_9$ représente un alcoyle en $C_1$ à $C_3$ ou un phényle, $R_{10}$ représente un alcoyle en $C_1$ à $C_4$ ou une mono- ou di-alcoyle en $C_1$ à $C_3$-amine et $R_{11}$ représente un alcoyle en $C_1$ à $C_3$ à substitution éventuelle, alcoxy en $C_1$ à $C_2$, où si $R_3$ = —$CH(CH_3)COOR_4$ le substituant $R_7$ peut également représenter un alcoyle en $C_1$ à $C_6$.

2. Agent selon la revendication 1, contenant comme matière active au moins un composé de formule I, où

$R_2$ est un hydrogène ou un méthyle,

$R_3$ = $-CH(CH_3)-COOR_4$ ou 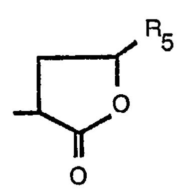

$R_4$ représente un alcoyle en $C_1$ à $C_3$ à substitution éventuelle, alcoxy en $C_1$ à $C_2$ ou encore un alcényle en $C_2$ à $C_4$ ou un alcynyle en $C_3$ à $C_4$,

$R_5$ est un hydrogène ou un méthyle et

$R_6$ représente un 2-furyle, un 2-tétrahydrofuryle ou $CH_2Z$,

Z représentant

a) —X—$R_7$, où X est un oxygène ou un soufre,

b) $—NH—N(R_8)(R_9)$,

c) $—OSO_2R_{10}$,

d) 1,2,4-triazole (1) et ses sels et complexes métalliques, où

$R_7$ si $R_3$ = lactone représente un allyle ou un propargyle, et

$R_7$ si $R_3 = —CH(CH_3)COOR_4$ représente au choix un allyle, un propargyle ou un alcoyle en $C_1$ à $C_3$,

$R_8$ et $R_9$ représentent de façon indépendante un alcoyle en $C_1$ à $C_2$ et

$R_{10}$ représente un alcoyle en $C_1$ à $C_2$ ou une monométhylamine.

3. Agent selon la revendication 1, contenant comme matière active au moins un composé de formule I, où $R_2$ est un hydrogène ou un méthyle,

$R_3$ = $-CH(CH_3)-COOR_4$ ou 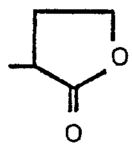

où

$R_4$ représente un alcoyle en $C_1$ à $C_3$,

$R_6$ représente un 2-furyle, 2-tétrahydrofuryle ou $—CH_2Z$, où

Z si $R_3 = —CH(CH_3)COOR_4$ représente au choix

a) $—OR_7$ avec $R_7$ = alcoyle en $C_1$ à $C_3$, ou

b) $—OSO_2R_{10}$, où $R_{10}$ = alcoyle en $C_1$ à $C_2$ ou monométhylamine, ou

c) 1,2,4-triazole, et où Z si $R_3$ = lactone a l'une des deux significations b) ou c) ci-dessus.

4. Agent selon la revendication 1, contenant au moins un des composés du groupe
N-(2-propyn-1-yloxyacétyl)-N-(2-méthylnaphtyl)-N-(2-oxo-tétrahydrofuran-3-yl)-amine,
N-(2-méthylnaphtyl)-N-(2-propyn-1-yl-oxyacétyl)-alanine-méthylester ou
N-(2-méthylnaphtyl)-N-(2-propyn-1-yl-oxyacétyl)-alanine-isopropylester comme matière active.

5. Agent selon la revendication 1 contenant au moins le composé N-(2-propén-1-yloxyacétyl)-N-(2-méthylnaphtyl)-N-(2-oxo-tétrahydrofuran-3-yl)-amine, comme matière active.

6. Agent selon la revendication 1 contenant au moins un des composés du groupe
N-(2-méthylnaphtyl)-N-méthoxyacétyl-alanine-2-méthoxyéthylester,
N-(2-méthylnaphtyl)-N-méthoxyacétyl-alanine-2-isopropylester,
N-(2,3-diméthylnaphtyl)-N-méthoxyacétyl-alanineméthylester comme matière active.

7. Agent selon la revendication 1, contenant au moins le composé
N-(2-méthylnaphtyl)-N-(isopropoxyacétyl)-alanineméthylester comme matière active.

8. Agent selon la revendication 1, contenant au moins le composé
N-(2,3-diméthylnaphtyl)-N-éthoxyacétyl-alanine-méthylester comme matière active.

9. Agent selon la revendication 1 contenant au moins le composé
N-(2,3-diméthylnaphtyl)-N-(β-méthoxy-éthoxyacétyl)-N-(2-oxo-tétrahydrofuran-3-yl)-amine comme matière active.

10. Agent selon la revendication 1 contenant au moins un des composés du groupe
N-(2-méthylnaphtyl)-N-méthylsulfonyloxyacétyl-alanine-méthylester
N-(2-méthylnaphtyl)-N-méthylsulfonyloxyacétyl-alanine-isopropylester,
N-méthylsulfonyloxyacétyl-N-(2-méthylnaphtyl)-N-(2-oxo-tétrahydrofuran-3-yl)-amine,
N-(2,3-diméthylnaphtyl)-N-(méthylsulfonyloxyacétyl)-N-(2-oxo-tétrahydrofuran-3-yl)-amine comme matière active.

11. Agent selon la revendication 1, contenant au moins un des composés du groupe
N-(2-méthylnaphtyl)-N-(N′-méthylsulfamoyloxyacétyl)-alanine-méthylester,
N-(2,3-diméthylnaphtyl)-N-(N′-méthylsulfamoyloxyacétyl)-alanine-méthylester,
N-(N′-méthylsulfamoyloxyacétyl)-N-(2-méthylnaphtyl)-N-(2-oxo-tétrahydrofuran-3-yl)-amine,
N-(2,3-diméthylnaphtyl)-N-(N′-méthylsulfamoyloxyacétyl)-N-(2-oxo-tétrahydrofuran-3-yl)-amine,
comme matière active.

12. Agent selon la revendication 1, contenant au moins un des composés du groupe
N-(2-méthylnaphtyl)-N-[1,2,4-triazolyl(1)-acétyl]-alanineméthylester,
N-(2-méthylnaphtyl)-N-[1,2,4-triazolyl(1)-acétyl]-alanine-2-méthoxyéthylester,
N-(2-méthylnaphtyl)-N-[1,2,4-triazolyl(1)-acétyl]-alanineisopropylester,
N-(2,3-diméthylnaphtyl)-N-[1,2,4-triazolyl(1)-acétyl]-alanineméthylester,
N-(2-méthylnaphtyl)-N-(2-oxo-tétrahydrofuran-3-yl)-N-[1,2,4-triazolyl(1)-acétyl]-amine,
n-(2-méthylnaphtyl)-N-(2-oxo-5-méthyl-tétrahydrofuran-3-yl)-N-[1,2,4-triazolyl(1)-acétyl]amine,
N-(2,3-diméthylnaphtyl)-N-(2-oxo-tétrahydrofuran-3-yl)-N-[1,2,4-triazolyl(1)-acétyl]-amine, comme matière active.

13. Agent selon la revendication 1, contenant au moins un des composés du groupe
N-(2-méthylnaphtyl)-N-(2-oxo-tétrahydrofuran-3-yl)-N-[tétrahydrofuran-3-yl)-N-[tétrahydrofuranoyl(2)]-amine,
N-(2,3-diméthylnaphtyl)-N-(2-oxo-tétrahydrofuran-3-yl)-N-[tétrahydrofuranoyl(2)]-amine,
N-(2-méthylnaphtyl)-N-[tétrahydrofuranoyl(2)]-alanine-méthylester,

N-(2-méthylnaphtyl)-N-[tétrahydrofuranoyl(2)]-alanine-3-méthoxyéthylester,
N-(2-méthylnaphtyl)-N-[tétrahydrofuranoyl(2)]-alanine-isopropylester,
N-(2,3-diméthylnaphtyl)-N-[tétrahydrofuranoyl(2)]-alanineméthylester, comme matière active.

14. Agent selon la revendication 1 contenant au moins un des composés du groupe
N-(2,3-diméthylnaphtyl)-N-[furanoyl(2)]-alanineméthylester,
N-(2-méthylnaphtyl)-N-[furanoyl(2)]-alanine-2-méthoxyéthylester, comme matière active.

15. Agent selon la revendication 1 contenant au moins le composé
N-(2-méthylnaphtyl)-N-(N'-2-phénylhydrazinoacétyl)-alanine-méthylester, comme matière active.

16. Agent selon la revendication 1 contenant au moins un des composés du groupe
N-(2-méthylnaphtyl)-N-(N',N'-2-diméthylhydrazinoacétyl)-alanineméthylester,
N-(2,3-diméthylnaphtyl-N-(N',N'-2-diméthylhydrazinoacétyl)-alanineméthylester, comme matière active.

17. Procédé de préparation d'alpha-naphtylamines N-acylées de formule I selon la revendication 1, caractérisé en ce que:

A. on acyle un composé de formule II avec un acide carboxylique de formule III ou son halogénure d'acide ou anhydride d'acide

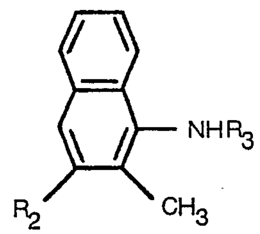

(II)

$HO-CO-R_6$ ,

(III)

ou

B. on fait réagir un composé de formule IV on son sel avec au choix un composé de formule V, VI ou VII,

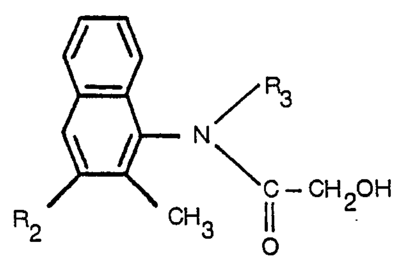

(IV)

\+

$Hal-SO_2R_{10}$ (V)
ou
$Hal-CO-R_{11}$ (VI)
ou
$Hal-R_7$ (VII)

ou

C. on fait réagir un composé de formule X au choix avec un composé de formule XI, XII, XIII ou XIIIa,

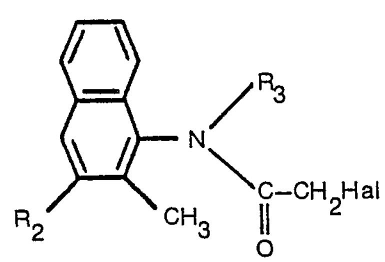

(X)

\+

$MXR_7$ (XI)
$MO(CO)R_{11}$ (XII)
$NH_2N(R_8)(R_9)$ (XIII)
M—Azol (XIIIa)

où dans les formules mentionnées ci-dessus les substituants $R_2$, $R_3$, $R_6$ à $R_{11}$ et X ont les significations données pour la formule I, tandis que "Hal" représente un halogène, "M" représente un hydrogène ou un cation métallique, et "azolyle" un 1,2-pyrazolyle(1) ou un 1,2,4-triazolyle(1).

18. Procédé selon la revendication 17, caractérisé en ce qu'on prépare un des composés de formule I selon l'une des revendications 2 à 16.

19. Application d'un composé de formule I de la revendication 1 pour combattre ou prévenir l'attaque des champignons sur les plantes.

20. Application d'un composé de formule I de la revendication 1 selon l'une des revendications 2 à 16, selon la revendication 19.